# EUROPÄISCHE PATENTSCHRIFT

(11) **EP 0 725 792 B1**
(45) Veröffentlichungstag und Bekanntmachung des Hinweises auf die Patenterteilung: **29.12.2004**
(21) Anmeldenummer: 94930216.0
(22) Anmeldetag: 25.10.1994
(51) Int. Cl.: C07K 14/22, C12N 5/10, C12N 15/63, C07K 16/12

(54) **VERFAHREN ZUR HERSTELLUNG REKOMBINANTER PilC-PROTEINE**
PROCESS FOR PRODUCING RECOMBINANT PilC PROTEINS
PROCEDE DE PREPARATION DES PROTEINES PilC RECOMBINANTES

(30) Priorität: 26.10.1993 DE 4336530
(43) Veröffentlichungstag der Anmeldung: 14.08.1996
(73) Patentinhaber: Max-Planck-Gesellschaft zur Förderung der Wissenschaften e.V., 80084 München (DE)
(72) Erfinder: MEYER, Thomas Franz Ferdinand, D-72076 Tübingen (DE); RUDEL,Thomas,The Scripps Research Institute,, La Jolla, California 92037 (US); RYLL, Roland Richard, D-72108 Rottenburg (DE); SCHEUERPFLUG, Ina Bärbel;MPI f.Infektionsbiologie, D-10117 Berlin (DE)
(74) Vertreter: VOSSIUS & PARTNER
(86) Internationale Anmeldenummer: PCT/EP1994/003494
(87) Internationale Veröffentlichungsnummer: WO 1995/011919

(56) Entgegenhaltungen:
- WO-A-92/13871
- WO-A-94/08013
- EMBO JOURNAL, Bd. 10, no.2, 1991 Seiten 477-488, A.B- JONSSON ET AL. 'Phase variation of gonococcal pili by frameshift mutation in pilC, a novel gene for pilus assembly'
- MOLECULAR MICROBIOLOGY, Bd. 6, no.22, 1992 Seiten 3439-3450, T. RUDEL ET AL. 'Interaction of two variable proteins (PilE and PilC) required for pilus-mediated adherence of N. gonorrhoeae to human epithelial cells'
- JOURNAL OF CHROMATOGRAPHY, Bd. 411, 1987 Seiten 177-184, E. HOCHULI ET AL. 'New metal chelate adsorbent selective for proteins and peptides containing neighbouring histidine residues'

## Beschreibung

Die Erfindung betrifft rekombinante Gensequenzen zur Synthese eines Proteins mit der biologischen Aktivität des PilC-Proteins. Ferner betrifft die Erfindung DNA-Rekombinations-Verfahren zur Herstellung von Proteinen mit der biologischen Aktivität des PilC-Proteins sowie die darin benötigten molekularbiologischen Werkzeuge. Außerdem betrifft die Erfindung Proteine mit der biologischen Aktivität des PilC-Proteins.

Ein entscheidender Schritt beim Zustandekommen und der Manifestation einer jeglichen Infektion ist die Bindung des Erregers an bestimmte molekulare Strukturen (Rezeptoren) des Wirtsorganismus. Die für die Bindung verantwortlichen Strukturen des Erregers werden hier als Adhäsine bezeichnet. Es ist möglich, daß multiple molekulare Wechselwirkungen zwischen Adhäsinen des Erregers und Rezeptoren des Wirtsorganismus für das Zustandekommen und/oder die Manifestation einer Infektion erforderlich sind. Andererseits ist denkbar, daß durch die Blockierung einer einzigen molekularen Wechselwirkung zwischen einem wichtigen Adhäsin und einem Rezeptor eine Infektion bereits verhindert wird.
Die Blockierung von molekularen Adhäsin-Rezeptor-Wechselwirkungen stellt eine mögliche Form der Prävention bzw. der Therapie von Infektionskrankheiten dar. Präventive Ansätze beinhalten beispielsweise die Bildung von Antikörpern, die spezifisch gegen das Adhäsin gerichtet sind und die Wechselwirkung mit seinem Rezeptor blockieren, durch aktive Immunisierung (Vakzinierung). Die Wechselwirkung kann aber im Sinne einer präventiven oder therapeutischen Maßnahme prinzipiell auch durch passiv verabreichte Antikörper oder andere Substanzen, beispielsweise adhäsin- oder rezeptoranaloge Substanzen, verhindert werden, die hier zusammenfassend als Inhibitoren bezeichnet werden. Wichtige Adhäsine zahlreicher gramnegativer Krankheitserreger sind die Pili (auch Fimbrien oder Fibrillen genannt). Es handelt sich um polymere Strukturen, die feine fadenförmige Anhängsel an der Bakterienoberfläche bilden. Die Pili vermitteln die Bindung der Bakterien an spezifische Rezeptoren des Wirtsorganismus über die in den Pili enthaltenen Adhäsine. In einigen Fällen wurde gezeigt, daß der Verlust der Pili zum Verlust der Infektiösität der Erreger führt. Dieser Verlust der Infektiösität wird durch den Verlust der Bindungsfähigkeit erklärt. Folglich kann durch Blockierung der molekularen Wechselwirkung zwischen Pilus-Adhäsin und Rezeptor die Infektion des Wirtsorganismus vermieden oder nachträglich unterbunden werden.

Bei gramnegativen Bakterien kennt man unterschiedliche Typen von Pili. Die Mehrzahl aller bekannten Pili sind heteropolymere Strukturen, die aus mehreren Untereinheiten bestehen, einer Hauptuntereinheit und weiteren, in nur wenigen Kopien vorhandenen Minder-Untereinheiten. In einigen gut untersuchten Fällen stellen die Minder-Untereinheiten die eigentlichen adhäsiven Strukturen (Adhäsine) dar, während die in hoher Kopienzahl vorliegende Hauptuntereinheit Gerüstfunktion übernimmt (Lindberg et al., Nature 328, 84-87, 1987).
Zahlreiche pathogene gramnegative Bakterienspezies bilden Typ 4-Pili, die auch als N-Me-Phe- oder Typ IV-Pili bezeichnet werden. Hierzu zählen die pathogenen Neisserien Spezies N. gonorrhoeae und N. meningitidis, die Gonorrhoe bzw. bakterielle Meningitis beim Menschen verursachen. Ein wirksamer Impfstoff gegen N. gonorrhoeae existiert aber bisher nicht. Dagegen sind Kapsel-spezifische Impfstoffe gegen einige Serogruppen von N. meningitidis erhältlich, die jedoch nur partiellen Schutz bieten und immunologisch als problematisch angesehen werden. Die gängige Behandlung der Infektionen erfolgt daher mit Antibiotika, wobei die ständig ansteigende Resistenzverbreitung Probleme aufwirft. Die Entwicklung alternativer Behandlungsmethoden ist somit dringlich. Ein anderer wichtiger Typ 4-Pili-bildender Erreger des Menschen ist Pseudomonas aeruginosa (Sastry et al., FEBS Letters 151, 253-255, 1983), der ein zentrales Problem bei Patienten mit zystischer Fibrose, bei immungeschwächten Patienten und im Zusammenhang mit septischen Infektionen darstellt. Wirksame Impfstoffe bzw. Inhibitoren gegen diesen Erreger sind noch nicht verfügbar; problematisch ist vor allem der Anstieg in der Verbreitung multiresistenter Stämme. Alternative Behandlungsmethoden sind daher auch hier dringend erforderlich. Als weitere Beispiele wichtiger Typ 4-Pili-bildender Krankheitserreger werden enteropathogene Escherichia coli (EPEC; Giron et al., Science 254, 710-713, 1991) , Vibrio cholerae (Shaw et al., Infect. Immun. 58, 3042-3049, 1990), Bakteroides nodosus (McKern et al., FEBS Letters 164, 149-153, 1983), Moraxella bovis (Marrs et al., J. Bacteriol. 163, 132-139, 1985) und andere Erreger genannt, die Krankheiten bei Mensch und Tier auslösen und gegen die ebenfalls intensiv nach einem Impfstoff gesucht wird.
Die Typ 4-Pili sind durch die Struktur ihrer Hauptuntereinheit definiert, die meist als Pilin bezeichnet wird; daneben existieren für einzelne Bakterienspezies spezifische Bezeichnungen für die Pilin-Hauptuntereinheit, z.B. PilE bei N. gonorrhoeae oder PilA bei Pseudomonas aeruginosa. Das Pilin der Typ 4-Pili unterscheidet sich in seiner Struktur grundlegend von Hauptuntereinheiten anderer Pilustypen, beispielsweise der Gruppe der Pap-ähnlichen Pili (Baga et al. J. Bacteriol. 157, 330-333, 1984). Merkmale des Pilins der Typ 4-Pili sind (a) die kurze, positiv geladene, aminoterminale Signalsequenz der Proform des Pilins (Ausnahme V. cholerae Typ 4-Pilin), (b) der hydrophobe aminoterminale Bereich des reifen Pilins, der starke Sequenzhomologie zwischen unterschiedlichen Typ 4-Pilinen aufweist, (c) die Modifikation des aminoterminalen Phe-Restes des reifen Pilins durch eine N-ständige Methylgruppe (N-Me-Phe), (d) zwei Cys-Reste im carboxyterminalen Bereich des Pilin, die einen Loop bilden und (e) eine Eigenschaft, die als "twitching motility" bezeichnet wird.

PilC-Proteine wurden bisher als Bestandteile von N. gonorrhoeae und N. meningitidis nachgewiesen. Als Funktion dieser PilC-Proteine wurde bisher eine Assembly-Funktion bei der Pilus-Biogenese angenommen (Jonsson, Dissertation, New Series No. 322, University of Umea, ISSN 0346-6612). Hinweise für eine mögliche Rolle als wichtiges Adhäsin erbrachten diese Arbeiten jedoch nicht. In anderen Versuchen gelang es, Mutanten von N. gonorrhoeae zu isolieren, die zwar noch. Pili bildeten aber keine Adhärenz mehr an Epithelzellen zeigten (Rudel et al., Mol. Microbiol. 6, 3439-3450, 1992). Diese Mutanten stellten sich als phasenvariante Stämme heraus, die die Bildung eines PilC-Proteins abgeschaltet hatten. Eine direkte Funktion der Neisserien PilC-Proteine konnte aus diesen Experimenten nicht abgeleitet werden. Die Experimente zeigten jedoch, daß das Assembly der Neisserien Pili auch in Abwesenheit von PilC-Proteinen erfolgen kann. Es gelang zuvor, ein einziges PilC-Protein codierendes Gen in E. coli zu clonieren. Dies wurde erreicht durch die gelelektrophoretische Reinigung von PilC-Protein aus isolierten Pili der Neisserien (Jonsson et al., EMBO J. 10, 477-488, 1991 WO9 213 871). Das gelelektrophoretisch gereinigte PilC-Protein wurde zur Gewinnung von Antiserum eingesetzt. Das gelelektrophoretisch gereinigte PilC-Protein besaß keine biologische Aktivität im Sinne dieser Erfindung. Das dagegen gerichtete Antiserum besitzt daher nicht die erfindungsgemäße Eigenschaft, die Bindung von piliierten Neisserien an Epithelzellen zu blockieren. Mit Hilfe des Antiserums gelang es zunächst, einen E. coli Phagenclon zu identifizieren, der ein partielles PilC-codierendes Gen trug. Mit Hilfe des partiellen PilC-codierenden Gens konnte durch DNA-Hybridisierung ein E. coli-Clon mit einem intakten PilC-codierenden Gen identifiziert werden (Jonsson et al. EMBO J. 10, 477-488, 1991 WO 9 213 871). Dieses rekombinante PilC-codierende Gen war aufgrund seiner variablen, homopolymeren Sequenz translationell inaktiv, so daß eine Synthese von biologisch aktivem PilC-Protein nicht erfolgte. Die Nucleotidsequenz dieses ersten PilC-codierenden Gens (pilC1) aus dem Stamm N. gonorrhoeae MS11 ist bekannt; ebenso ist eine partielle Sequenz eines zweiten Gens (pilC2) aus diesem Stamm bekannt (Jonsson et al., EMBO J. 10, 477-488, 1991; Jonsson, Dissertation, New Series No. 322, University of Umea, ISSN 0346-6612, WO 9 213 871). Mit keinem dieser Gene konnte jedoch bisher PilC hergestellt werden.

Somit liegt der Erfindung im wesentlichen das technische Problem zugrunde, biologisch aktive Proteine mit der biologischen Aktivität des PilC-Proteins bereitzustellen.
Die Lösung dieses technischen Problems wird erzielt durch die Bereitstellung der in den Patentansprüchen gekennzeichneten Ausführungsformen.

Somit betrifft die Erfindung rekombinante Gensequenzen zur Synthese eines Proteins mit der biologischen Aktivität des PilC-Proteins, deren phasenvariabler Signalpeptidcodierender, eine homopolymere Nucleotidsequenz enthaltender Sequenzabschnitt durch die folgende Veränderungen charakterisiert ist,
(a) Modifikation der homopolymeren Nucleotidsequenz zu einer invariablen heteropolymeren Nucleotidsequenz oder so daB die Expression der rekombinanten Gensequenz in einer Wirtszelle unbeeinflußt von Phasenvariation ermöglicht wird.

Diese erfindungsgemäßen DNA-Sequenzen dienen der Expression von Proteinen mit der biologischen Aktivität des PilC-Proteins.
Der Begriff "biologische Aktivität des PilC-Proteins" betrifft erfindungsgemäß die Fähigkeit des codierten Proteins das Assembly von Typ 4-Pili zu unterstützen, die Bindung von Typ 4-Pilus-tragenden Bakterien an zelluläre Rezeptoren zu vermitteln oder die immunologische Eignung zur Induktion von Antikörpern gegen Typ 4-Pilus-tragende Bakterien, die mit der Anlagerung der Bakterien an ihre zellulären Rezeptoren kompetieren und vorzugsweise die Anlagerung blockieren.
Erfindungsgemäß betrifft der Begriff "Protein" natürlich vorkommende Proteine oder Modifikationen oder Fragmente davon mit der genannten biologischen Aktivität.
Mit dem erfindungsgemäß verwendeten Begriff "PATHOGENE, TYP 4-PILI-TRAGENDE BAKTERIEN" werden Bakterien bezeichnet, die einerseits ursächlich mit der Pathogenese von Krankheiten in Zusammenhang stehen und andererseits als ein wesentliches Element ihrer pathogenen Eigenschaft Typ 4-Pili bilden, die für die Bindung der Bakterien an zelluläre Rezeptoren im infizierten Wirtsorganismus notwendig sind.

"HOMOPOLYMER" sind Nucleotidsequenzen, die aus einer Abfolge identischer Nucleotide bestehen (z.B. 5'-CCCCCCC-3'). Erfindungsgemäß werden "HOMOPOLYMERE NUCLEOTIDSEQUENZEN" durch ihre Eigenschaft definiert, spontan oder induziert ein oder mehrere identische Nucleotide zu der vorhandenen homopolymeren Nucleotidsequenz hinzufügen oder aus ihr zu verlieren. Der Gewinn bzw. Verlust identischer Nucleotide einer homopolymeren Nucleotidsequenz bewirkt eine "PHASENVARIATION". Diese Phasenvariation wird durch einen RecA-Protein unabhängigen Prozess bewirkt, der im wesentlichen spontan abläuft (Robertson & Meyer, Trends Genet., 8, 422-427, 1992). Die der Phasenvariation zugrundeliegenden Veränderungen in der Nucleotidsequenz wirken sich auf den translationellen Leserahmen eines Gens und folglich auf die Bildung des intakten Genprodukts aus. Im Sinne dieser Erfindung ist Phasenvariation unerwünscht, weil Phasenvariation der PilC-codierenden DNA unter der Kontrolle eines starken Promotors zur Veränderung des Leserahmens und damit zum Verlust der Bildung des gewünschten Proteins mit der biologischen Aktivität de PilC-Proteins führt. Eine "INVARIABLE HETEROPOLYMERE NUCLEOTIDSEQUENZ" im Sinne der Erfindung ist daher eine Nucleotidsequenz, die aus einer homopolymeren Nucleotidsequenz durch Einfügen oder Austausch nichtidentischer Nucleotide (im Falle. der Sequenz 5'-GGGGGGGGGGGGG-3' also der Nucleotide A, C und/oder T) hervorgegangen und aufgrund dieser Veränderungen "NICHT PHASENVARIABEL" geworden ist, d.h. keine Phasenvariation zeigt. Es ist aufgrund des genetischen Codes möglich, die homopolymere Nucleotidsequenz so zu modifizieren, daß die codierte Aminosäuresequenz-entweder unverändert bleibt oder ebenfalls verändert wird.

Ein "SIGNALPEPTID" im Sinne dieser Erfindung stellt eine Aminosäuresequenz am Aminoterminus der Proform eines vom gramnegativen Bakterien sekretierten Proteins dar. Ein Signalpeptid ermöglicht die Sekretion eines Proteins über den generellen Exportweg der inneren bakteriellen Membran (Pugsley, Microbiol. Rev., 57, 50-108, 1993). Der phasenvariable Signalpeptid-codierende Abschnitt der erfindungsgemäßen Gensequenz ist durch Modifikation seiner homopolymeren Nucleotidsequenz (siehe oben) verändert. Die Veränderung (Modifikation wird mit dem Ziel eingeführt, die Expression der Gensequenz in einer Wirtszelle unbeeinflußt von Phasenvariation zu ermöglichen, um so die Bildung von PilC-Protein unter stabilen Bedingungen und in großen Mengen (Überproduktion) zu gewährleisten. Hybridisierung von chromosomaler DNA von beispielsweise N. gonorrhoeae mit einem gesamten PilC-Gen als Probe ergeben Hinweise auf die Existenz zweier verwandter PilC-Gene im Chromosom dieser Spezies. Benutzt man jedoch subgenische Fragmente eines PilC-Gens als Probe, so können darüber hinaus weitere kreuzhybridisierende Gene sichtbar gemacht werden, die ebenfalls PilC-Gene darstellen, die allerdings nur entfernt verwandt sind. Der Nachweis solcher entfernt-verwandter PilC-Gene beruht auf der Kreuzhybridisierung konstanter Bereiche der DNA-Sequenz zwischen zwei oder mehr verwandten PilC-Genen. Konstante Bereiche der DNA-Sequenz lassen sich durch einen Sequenzvergleich zwischen zwei verwandten Genen definieren. Durch die Definition konstanter Bereiche der DNA-Sequenz ergibt sich daher eine Möglichkeit, auch entfernt-verwandte PilC-Gene zu identifizieren. Der erneute Sequenzvergleich mit solch einem entfernt-verwandten Gen ergibt wiederum neue konstante Bereiche der DNA-Sequenz, die erneut zur Identifizierung von PilC-Genen herangezogen werden können usw. Damit gelingt es schrittweise, alle Mitglieder der PilC-Genfamilie innerhalb und außerhalb einer Bakterien-Spezies zu erfassen.

Die erfindungsgemäßen rekombinanten Gensequenzen ermöglichen die Herstellung von Proteinen mit der biologischen Aktivität des PilC-Proteins in nennenswerten Mengen. Damit wird die allgemeine Identifizierung von Minder-Untereinheiten der Typ 4-Pili und deren zuverlässige Charakterisierung als Adhäsine ermöglicht. Die Erfindung gestattet insbesondere den Nachweis der PilC-Proteine von pathogenen Neisserien (N. gonorrhoeae und N. meningitidis) in ihrer Funktion als wichtige Adhäsine. Ferner gestattet die Erfindung den Nachweis PilC-analoger Proteine anderer Typ 4-Pili-bildender Bakterienspezies. Der erfindungsgemäße Nachweis der Adhäsin-Funktion einer Minder-Untereinheit eines Typ 4-Pilus bzw. PilC-analoger Proteine wurde bisher noch für keine Typ 4-Pili-produzierende Bakterienspezies beschrieben. Der Begriff "PilC-analoges Protein" bezieht sich dabei ausschließlich auf die strukturelle Verwandtschaft (von Nucleotid und Aminosäure-Sequenz) mit den PilC-Proteinen der pathogenen Neisserien und auf die analoge Funktion mit den Neisserien-PilC-Proteinen als rezeptorbindende Adhäsine. Die erfindungsgemäßen PilC-analogen Proteine von anderen Typ 4-Pili-bildenden Bakterienspezies sind nicht notwendigerweise mit den bereits in der Literatur als PilC bezeichneten Proteinen identisch oder verwandt (beispielsweise handelt es sich bei dem bekannten PilC-Protein von Pseudomonas aeruginosa (Nunn et al., J. Bacteriol. 172, 2911-2919, 1990) nicht um ein PilC-analoges Protein im Sinne dieser Erfindung).

Irrtümlicherweise wurde in einigen Fällen die Hauptuntereinheit der Typ 4-Pili als wichtiges Adhäsin eingestuft (Rothbard et al., PNAS (USA) 82, 919, 1985; Paranchych, In: The Bacteria Vol. XI, Molecular Basis of Bacterial Pathogenesis, Academic Press, 61-78, 1990 und Zitate darin). Aufgrund dieser Befunde bzw. Hypothesen wurden zahlreiche Versuche unternommen, die Adhärenz von Bakterien an menschliche oder tierische Zellen durch Antikörper bzw. Adhärenz-Hemmer zu blockieren oder durch Vakzinierung mit der Typ 4-Pilus-Hauptuntereinheit eine bakterielle Infektion zu unterbinden. Trotz partieller Erfolge (Paranchych, In: The Bacteria Vol. XI, Molecular Basis of Bacterial Pathogenesis, Academic Press, 61-78, 1990 und Zitate darin; Tramont, Clin. Microbiol. Rev. 2, S74-S77, 1989 und Zitate darin) gelang es aber bisher nicht, einen breit-wirksamen Impfstoff oder einen breit-wirksamen Inhibitor basierend auf der Hauptuntereinheit der Typ 4-Pili zu entwickeln. Hierfür gibt es zwei mögliche Erklärungen: (a) Die Hauptuntereinheit eines Typ 4-Pilus beinhaltet keine wichtigen Adhärenzfunktionen, so daß die Bindung der Pili an. den Rezeptor nicht blockiert wird und/oder (b) die Vakzine oder der Inhibitor sind aufgrund der strukturellen Variabilität der Pilin-Hauptuntereinheit nicht wirksam oder nicht breitwirksam.
Die letztgenannte Erklärung bezieht sich auf die Annahme, daß eine gegen Pilin gerichtete Vakzine zu einem Verlust der Adhärenzfähigkeit der Pili insgesamt führen könnte, unabhängig davon, ob Pilin ein Adhäsin darstellt oder nicht. Denn die Wirkung der gegen Pilin gerichteten Antikörper könnte die Strukturfunktion des Pilin und damit indirekt die Adhärenzeigenschaften der Pili insgesamt beeinträchtigen. Daß dieser Weg offensichtlich nicht zum Erfolg führt (Johnson et al., J. Infect. Dis. 163, 128-134, 1991), beruht weitgehend auf der erwähnten strukturellen Variabilität des Pilin. Es ist bekannt, daß Pilin von Typ 4-Pilus-bildenden Bakterienspezies inter-stammspezifische und/oder intrastammspezifische strukturelle Variabilität aufweist. Diese strukturelle Variabilität bedingt, daß eine indirekte Blockierung der Adhärenz mittels Antikörpern, die gegen die variable Pili-Hauptuntereinheit gerichtet sind, nicht breit wirksam ist, sondern auf den bestimmten Stamm oder die Variante beschränkt ist. Daher konnte auf der Basis der Pili-Hauptuntereinheit (Pilin) eine breit-wirksame Vakzine bisher nicht entwickelt werden.

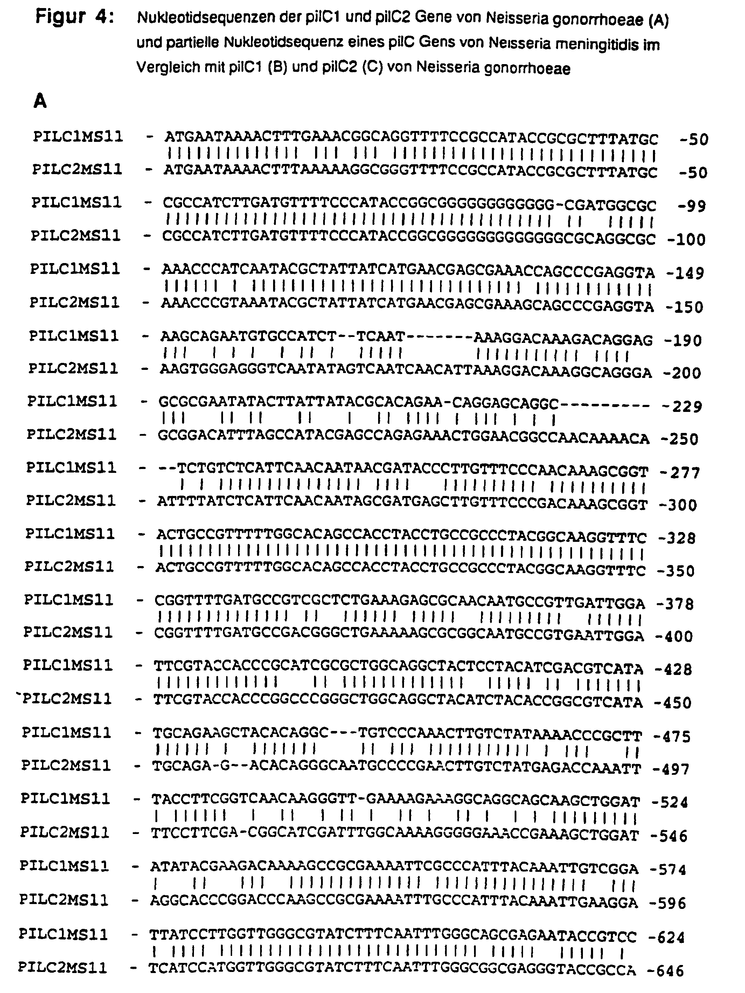

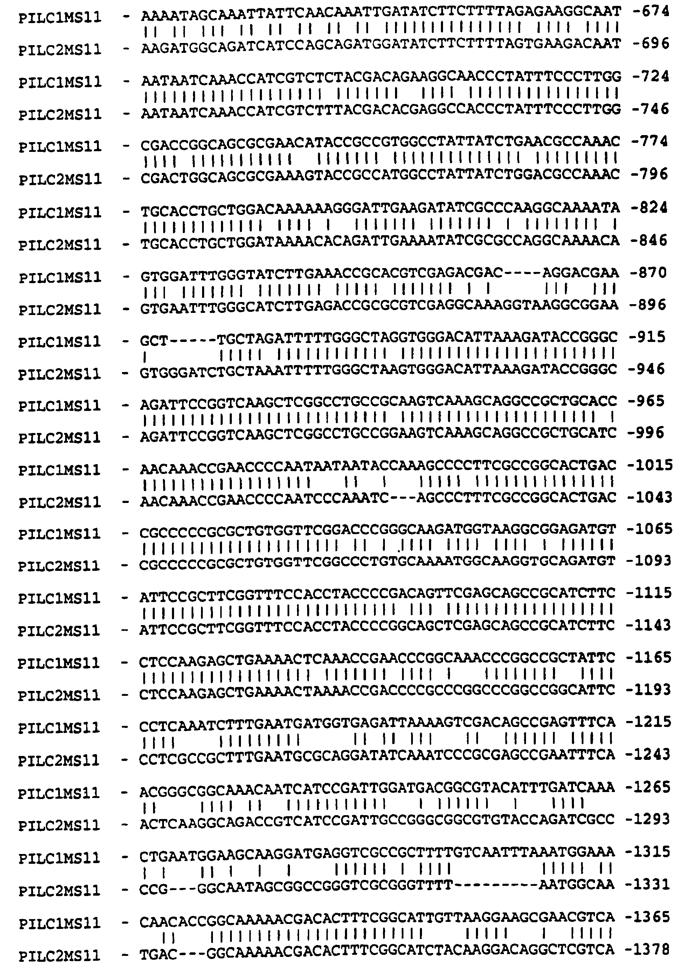

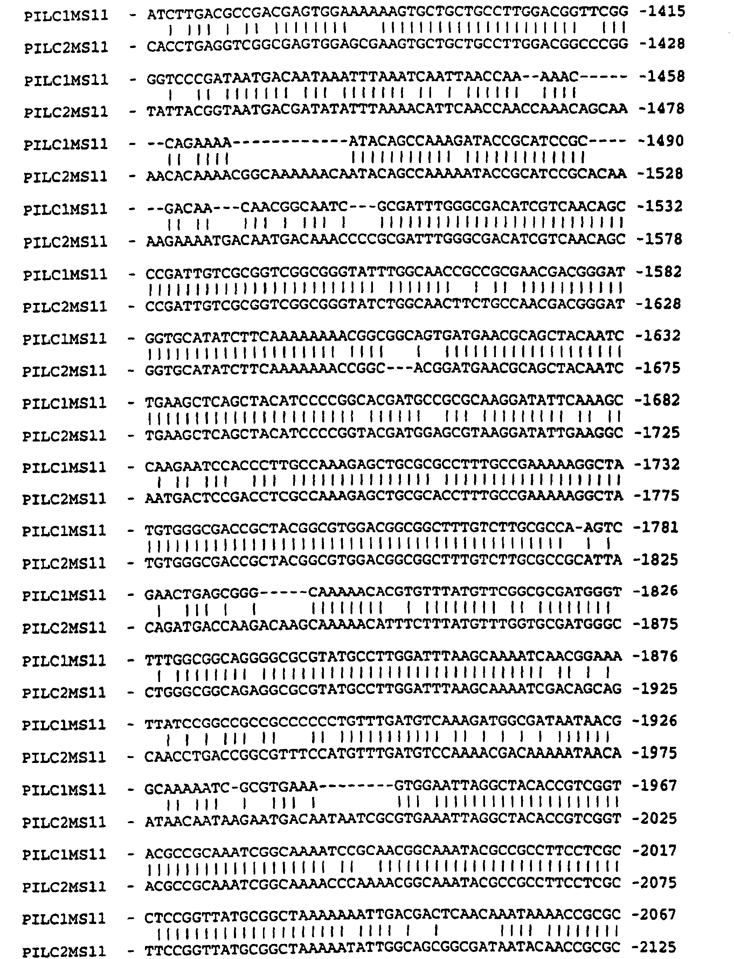

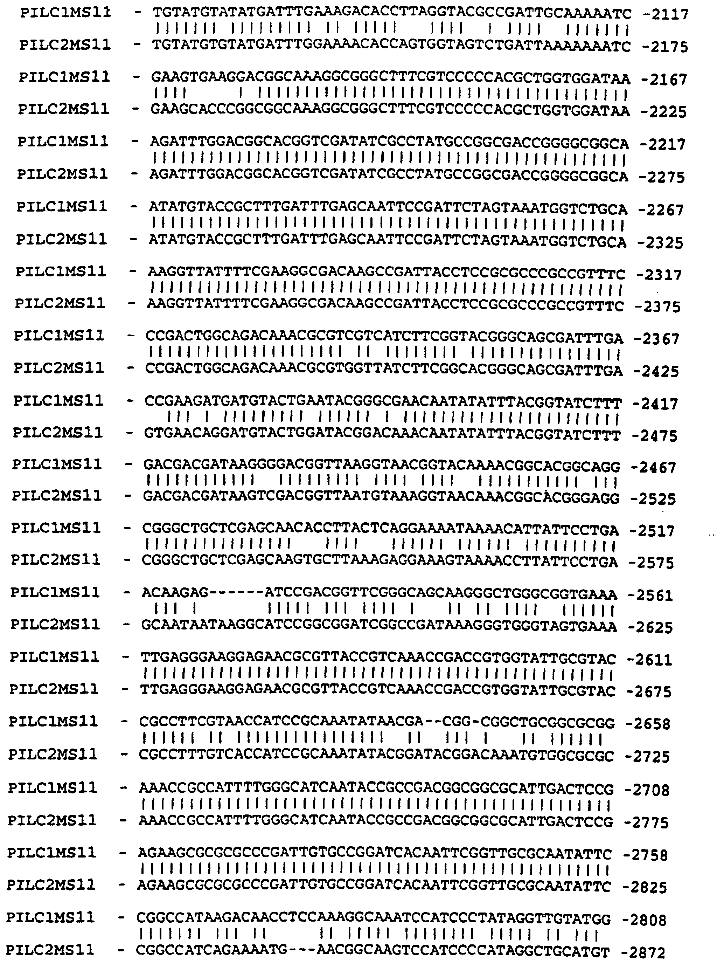

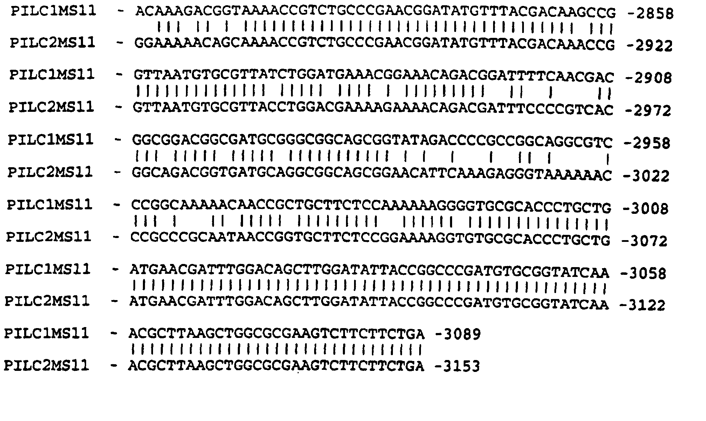

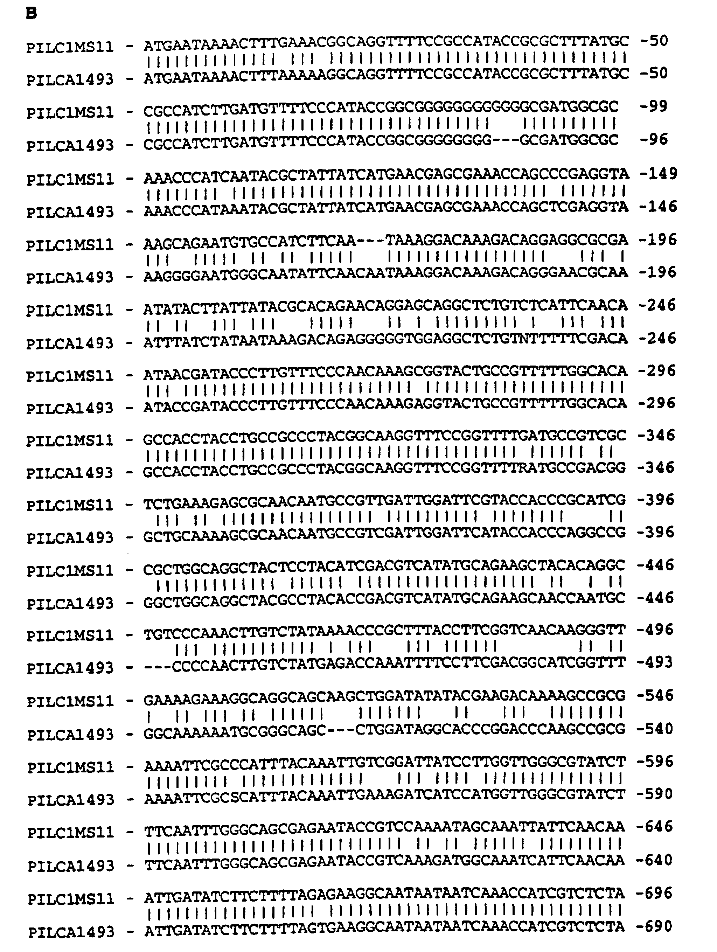

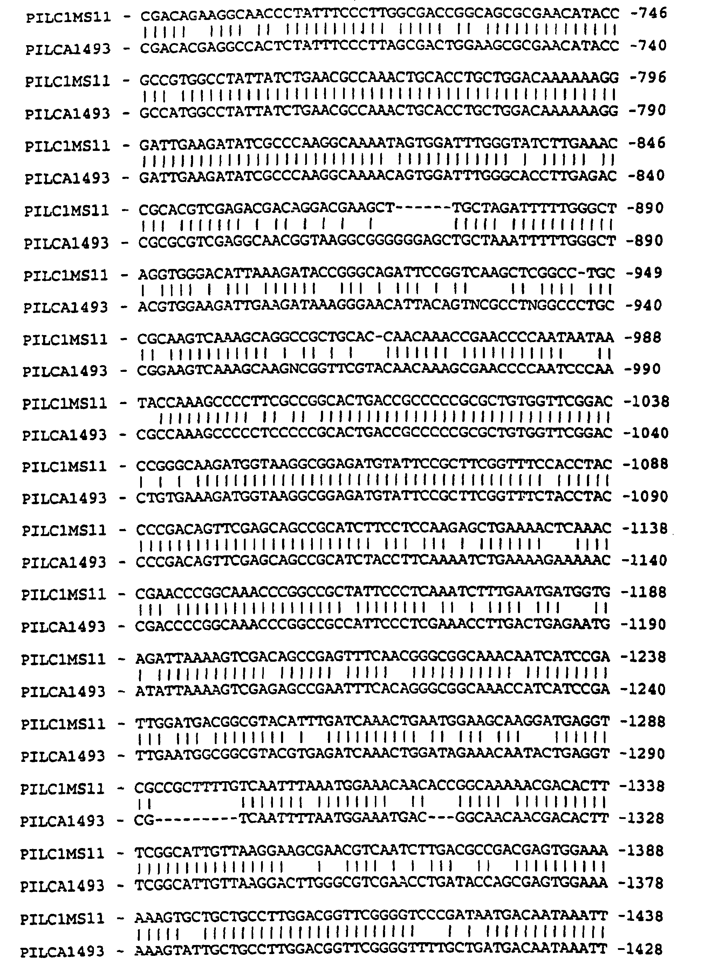

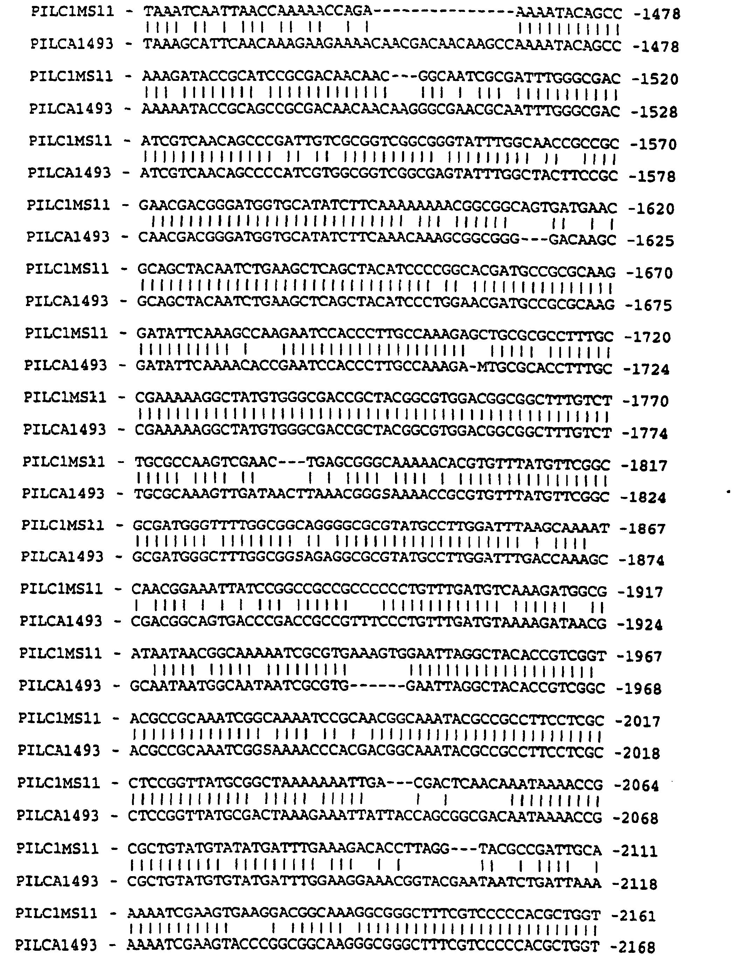

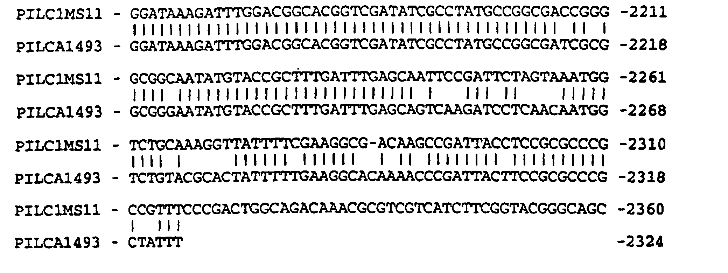

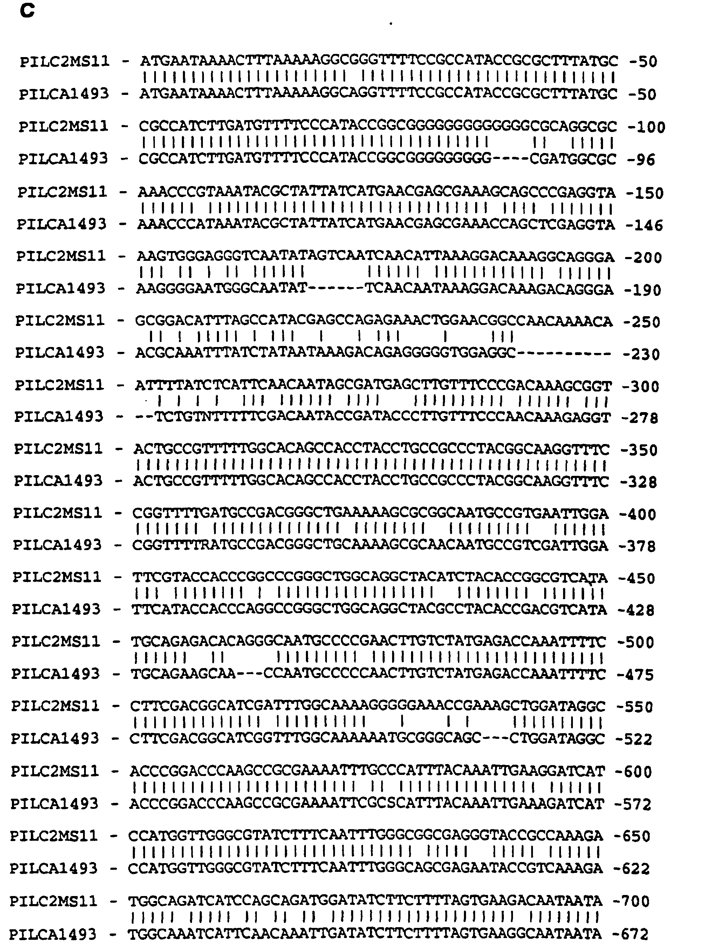

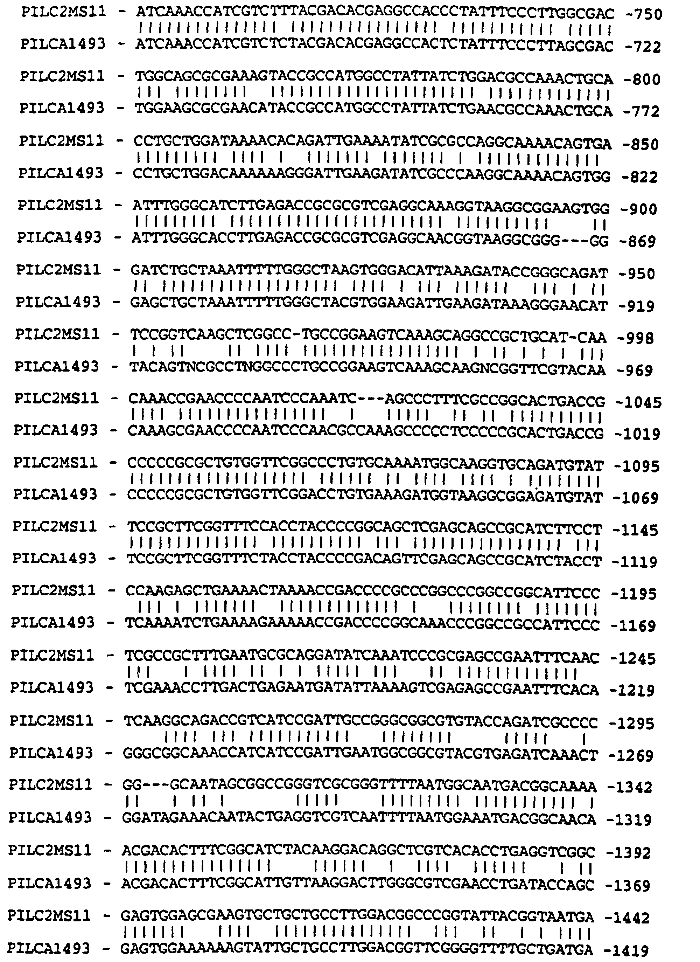

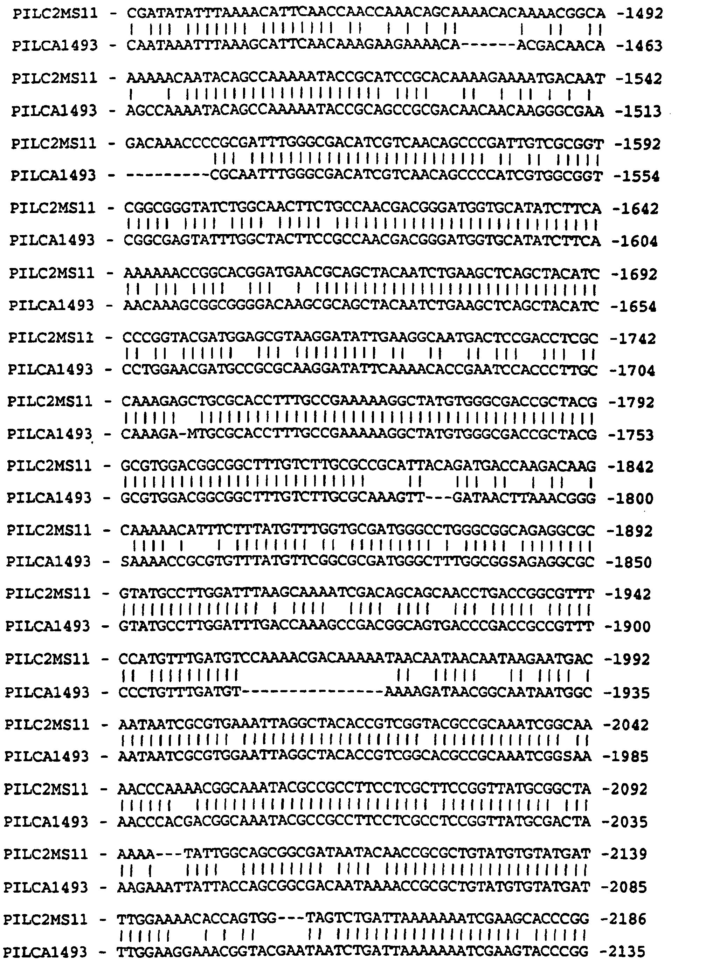

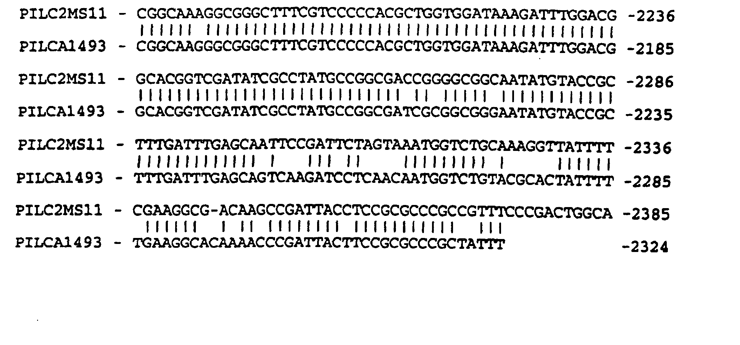

Die nun mit dem erfindungsgemäßen Gensequenzen herstellbaren PilC-Proteine, z.B. die von pathogenen Neisserien, besitzen ebenfalls eine strukturelle Variabilität, wie beispielsweise der Vergleich der codierenden Nucleotidsequenzen zeigt (Fig. 4). Im Gegensatz zur Variabilität des Pilins ist die Variabilität der PilC-Adhäsine jedoch deutlich geringer und wirkt sich nicht (oder in einem nicht bedeutsamen Ausmaß) auf die Funktion der PilC-Proteine aus, nämlich die molekulare Wechselwirkung mit dem Rezeptor. Diese Aussage wird durch die Kompetitionsexperimente mit biologisch aktivem PilC-Protein belegt (Tab. 2), da mit demselben PilC-Protein Bakterien, die unterschiedliche Pilin-Moleküle und/oder PilC-Proteine bilden, verdrängt werden können. Innerhalb einer Spezies werden daher zwar strukturell Variante Pilc-Proteine gebildet, diese PilC-Proteine einer Spezies erkennen jedoch dieselben oder nur wenige unterschiedliche Rezeptoren im Wirtsorganismus. Es ist daher möglich, auf der Basis von nur wenigen varianten Formen der PilC-Proteine einer Spezies eine breit-wirksame Vakzine bzw. breit-wirksame Adhärenz-Hemmer zu entwickeln. Gleichfalls ermöglicht es die Erfindung, auf der Basis von nur einem oder wenigen Rezeptoren breitwirksame Rezeptor-Analoga zu entwickeln.
Diese Möglichkeiten treffen auch für die PilC-analogen Adhäsine zu, die von Typ 4-Pili-bildenden Bakterien, die nicht der Gattung Neisseria angehören, gebildet werden. Denn es kann angenommen werden, daß der Zell-, Gewebs- und Wirtstropismus einer Spezies in weitem Maße durch die Wechselwirkung der Adhäsine mit ihren korrespondierenden zellulären Rezeptoren bestimmt wird. Diese Tropismen sind bei vielen Krankheitserregern, insbesondere auch bei Typ 4-Pilus-bildenden Bakterien, sehr ausgeprägt. Dieser Tatsache kann entnommen werden, daß auch die erfindungsgemäßen PilC-analogen Adhäsine einer jeweiligen Spezies mit ihren definierten Tropismen nur mit ein oder wenigen zell-, gewebs- und wirtsspezifischen Rezeptoren wechselwirken. Damit ergibt sich die Möglichkeit, entsprechend der Verwendung der PilC-Adhäsine der Neisserien, auf der Basis der PilC-analogen Adhäsine anderer Spezies bzw. deren Rezeptoren breitwirksame Vakzine und andere Inhibitoren einer Infektion zu entwickeln.

Ein wichtiger Aspekt der vorliegenden Erfindung war es, den Nachweis für die Funktion der PilC-Proteine als wichtige Adhäsine im Zusammenhang mit dem Zustandekommen einer Infektion zu führen. Hierzu war es nötig, biologisch aktives PilC-Protein in reiner Form zu gewinnen und seine biologische Aktivität in geeigneten Testsystemen nachzuweisen.

Zur Herstellung einer erfindungsgemäßen rekombinanten Gensequenz wurde das komplette pilC2-Gen aus dem Stamm N. gonorrhoeae MS11 auf Grundlage der über PilCl und dessen Gen bekannten Informationen isoliert und in E. coli cloniert. Um. mit diesem Gen das erfindungsgemäße Protein mit der biologischen Aktivität eines PilC-Proteins der Gattung Neisseria zu erzeugen, wurde der phasenvariable, Signalpeptid-codierende, homopolymere Sequenzabschnitt des pilC2-Gens so verändert, daß die Expression der. rekombinanten Gene in einer Wirtszelle unbeeinflußt von Phasenvariation ermöglicht wird. Dies wurde durch die Modifikation des homopolymeren Sequenzabschnitts zu einer heteropolymeren Sequenz unter Verwendung geeigneter Oligonucleotide mittels der Polymerase-Chain-Reaction (PCR) erreicht (Beispiel 2).

In einer bevorzugten Ausführungsform ist die erfindungsgemäße Gensequenz erhältlich durch Modifikation einer aus einem pathogenen, Typ 4-Pilus-tragenden Bakterium stammenden DNA-Sequenz. Diese Bakteriengruppe wurde bereits vorstehend näher charakterisiert. Beispiele solcher Bakterien sind Neisserien, insbesondere N. gonorrhoeae und N. meningitidis, Pseudomonaden, insbesondere P. aeruginosa, enteropathogene Escherichien, insbesondere E. coli (EPEC), Vibrio cholerae, Bakteroides nodosus und Moraxella bovis.

In einer besonders bevorzugten erfindungsgemäßen Ausführungsform ist die Gensequenz durch Modifikation einer DNA-Sequenz aus einem Bakterium der Gattung Neisseria, vorzugsweise Neisseria gonorrhoeae oder Neisseria meningitidis erhältlich.

Erfindungsgemäß werden die in der Figur 4 dargestellten Gensequenzen des N. gonorrhoeae pilC2 Gens und des N. meningitidis pilC A1493 Gens besonders bevorzugt. Die dargestellten Sequenzen beginnen bei Position 1 mit dem für die erste Aminosäure (Met) codierenden Codon ATG. Der für das Signalpeptid codierende Bereich erstreckt sich von Position 1 bis 99 bezogen auf das Gen pilC2. Darin enthalten ist der phasenvariable homopolymere Sequenzabschnitt, der sich von Position 79 bis 91 erstreckt. Die konstanten Nucleotide der gezeigten Sequenzen sind mit Sternen markiert. Einzelne konstante Sequenzabschnitte der Gene pilC1 und pilC2 sind in Tabelle 3 gesondert dargestellt.

DNA-Hybridisierungsexperimente mit dem kompletten pilC1-Gen, mit Fragmenten des pilC1-Gens, die mittels Restriktionendonucleasen gewonnen wurden, oder mit dem bisher bekannten Genabschnitt des pilC2-Gens von N. gonorrhoeae MS11 ergaben Hinweise auf die Existenz von 2 (Jonsson et al., EMBO J. 10, 477-488, 1991) bzw. maximal 3 (Bihlmaier et al., Mol. Microbiol. 5, 2529-2539, 1991) verwandten pilC-Genen innerhalb dieses Stammes. Die Bestimmung der Nucleotidsequenz des erfindungsgemäßen zweiten kompletten pilC-Gens (pilC2) und der Vergleich der beiden pilC-Nucleotidsequenzen (Fig. 4) ermöglichen es erfindungsgemäß, konservierte Bereiche der pilC-Gene festzulegen (Tabelle 3). Entsprechende subgenische, konservierte Fragmente bzw. Oligonucleotide eines PilC-Gens können dann, im Gegensatz zu kompletten Genen bzw. größeren Genfragmenten, durch ihre entsprechenden Homologien zu den konservierten Nucleotidsequenzen verwandter Gene als Hybridisierungsproben verwendet werden, um solche verwandten Gene insgesamt zu identifizieren und zu isolieren.
Somit ist die erfindungsgemäße Gensequenz in einer anderen Ausführungsform erhältlich durch Modifikation einer DNA-Sequenz, die mit einem konstanten Bereich der DNA-Sequenz der Figur 4 bzw. der Tabelle 3 hybridisiert und ein Protein mit der biologischen Aktivität des PilC-Proteins codiert.

Erfindungsgemäß beträgt die Länge der zu einer heteropolymeren Nucleotidsequenz modifizierten homopolymeren Nucleotidsequenz mindestens 5 Nucleotide. In den in Figur 4 angegebenen Sequenz-Beispielen handelt es sich um 12 G (pilC1), 13 G (pilC2), 9 G (pilC A1493); vgl. Position 79 bis Position 91 bezogen auf die Gensequenz pilC2.

In einer weiteren erfindungsgemäß bevorzugten Ausführungsform codiert die modifizierte Gensequenz ein Protein mit der biologischen Aktivität des PilC-Proteins, das einen zu seiner Reinigung geeigneten Oligo-Histidin-Bereich aufweist. Vorzugsweise enthält dieser Oligo-Histidin-Bereich 6 Histidin-Reste (His₆). Die Gegenwart des His₆-Peptids ermöglicht die selektive Bindung des erfindungsgemäßen PilC-Proteins an eine Nickel-NTA (Ni-Nitrilotriessigsäure) -Agarose-Säule (Hochuli et al. , J. Chromat., 411, 177-184, 1987; Hochuli et al., Bio/Techno. 6, 1321-1325, 1988), wobei das eluierte Protein reines PilC-Protein darstellt.

In einer erfindungsgemäß besonders bevorzugten Ausführungsform befindet sich der Oligo-Histidin-Bereich am N-Terminus oder am C-Terminus der reifen Form des codierten Proteins. Dadurch wird eine störungsfreiere räumliche Struktur des Proteins gewährleistet.

In einer anderen Ausführungsform betrifft die Erfindung rekombinante Vektoren, die eine erfindungsgemäße Gensequenz enthalten. Beispiele solcher Vektoren sind die in E. coli replizierenden Vektoren pBR322 und pBA, Vektoren, die auf den Bakteriophagen M13, fd oder lambda beruhen, Broad-Host-Range-Vektoren, Shuttle-Vektoren entsprechend den Hermes-Vektoren (Kupsch et al., zur Veröffentlichung eingereicht), die einen Einbau clonierter Gene in die DNA der rezipienten Neisseria-Zelle ermöglichen sowie das Plasmid ptetM25.2, das zur conjugativen Übertragung von Genen zwischen Neisserien benutzt werden kann (Kupsch et al., zur Veröffentlichung eingereicht).

In einer bevorzugten Ausführungsform der erfindungsgemäßen Vektoren steht die genannte Gensequenz unter der Kontrolle eines Promotors. Beispiele erfindungsgemäß geeigneter Promotoren sind Promotoren, die in gramnegativen Bakterien funktionell sind und insbesondere induzierbare Promotoren. In einer erfindungsgemäß besonders bevorzugten Ausführungsform ist der Promotor Ptrc, der in Gegenwart eines exprimierten lacl^{q} Gens in Neisseria und E. coli sowohl reprimiert als auch in Gegenwart von IPTG induziert werden kann.

In einer weiteren Ausführungsform betrifft die Erfindung Wirtszellen, die einen oder mehrere erfindungsgemäße rekombinante Vektoren enthalten.
Die erfindungsgemäßen Wirtszellen dienen der Replikation, Transkription und Translation der erfindungsgemäßen Gensequenz für die Synthese eines Proteins mit der biologischen Aktivität des PilC-Proteins dar. Es handelt sich vorzugsweise um gramnegative Bakterien, die es ermöglichen, das synthetisierte Protein über die innere Membran zu sekretieren und korrekt zu falten. Beispiele solcher Wirtszellen sind E. coli K12 und andere gramnegative Bakterien, für die geeigneten Clonierungsvektoren zur Verfügung stehen. Vorzugsweise ist die erfindungsgemäße Wirtszelle ein nicht-pilliierter Neisseria-Stamm, der - wie N. gonorrhoeae N174 - die Bildung seiner Pili aufgrund eines defekten pilE-Gens verloren hat. Das pilE-Gen codiert für die Hauptuntereinheit (Pilin) der Neisseria-Pili. Aufgrund des defekten pilE-Gens wird kein Pilin synthetisiert, was die Gewinnung von biologisch aktivem PilC-Protein ermöglicht, das absolut frei von Pilin ist.

In einer weiteren Ausführungsform betrifft die Erfindung Verfahren zur Herstellung eines im wesentlichen reinen Proteins mit der biologischen Aktivität des PilC-Proteins, bei dem man eine erfindungsgemäße Wirtszelle unter geeigneten Bedingungen züchtet und das Protein reinigt.

In einer bevorzugten Ausführungsförm des erfindungsgemäßen Herstellungsverfahrens erfolgt die Reinigung des Proteins mit der biologischen Aktivität des PilC-Proteins durch eine Affinitätschromatographie, vorzugsweise eine Affinitätschromatographie, bei der die im erfindungsgemäßen Protein enthaltenen Oligo-Histidin-Bereiche zur Bindung des Proteins benutzt werden.

Die Figuren zeigen:

### Figur 1:

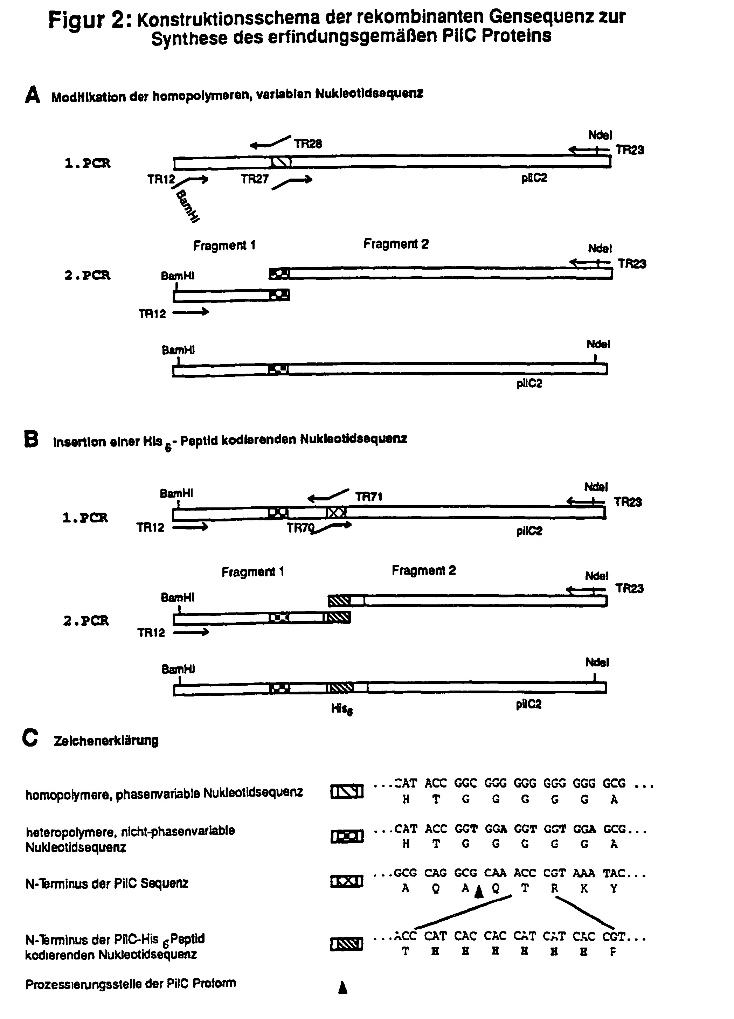

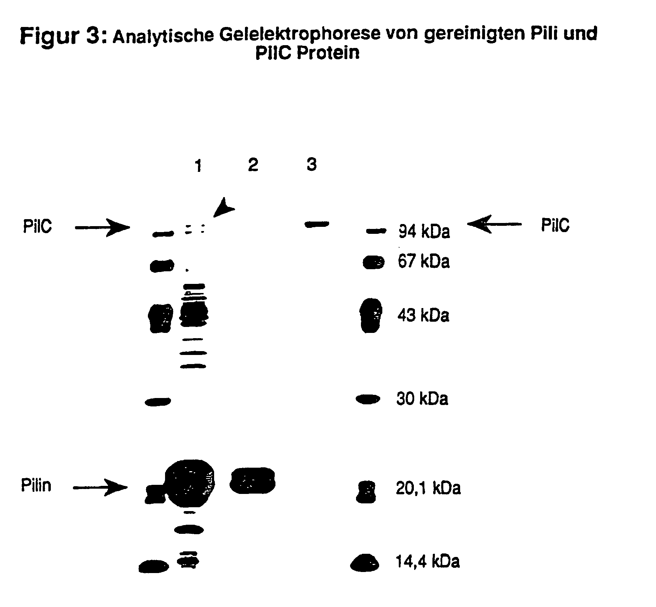

Restriktionskarten der Plasmide pTR27 und plS26. Das Plasmid pTR27 beinhaltet das natürliche pilC2-Gen des N. gonorrhoeae Stammes MS11-N133. Ausgehend von diesem Plasmid wurde in mehreren Schritten eine erfindungsgemäße Gensequenz erzeugt (Beispiel 2; Figur 2), die in Plasmid plS26 cloniert. vorliegt. Bei Plasmid plS26 handelt es sich um ein Hermes-. Konstrukt (Kupsch et al., zur Veröffentlichung eingereicht), das sowohl die Replikation in E. coli ermöglicht, als auch den Einbau der erfindungsgemäßen rekombinanten Gensequenz in das conjugative ptetM25.2 Plasmid in N. gonorrhoeae ermöglicht. Dieser Einbau erfolgt durch Übertragung der in. der "Shuttle Box" enthaltenen Gensequenz mittels doppelter: homologer Rekombination in den beiden gepunkteten Bereichen von plS26 mit ptetM25.2.

### Figur 2:

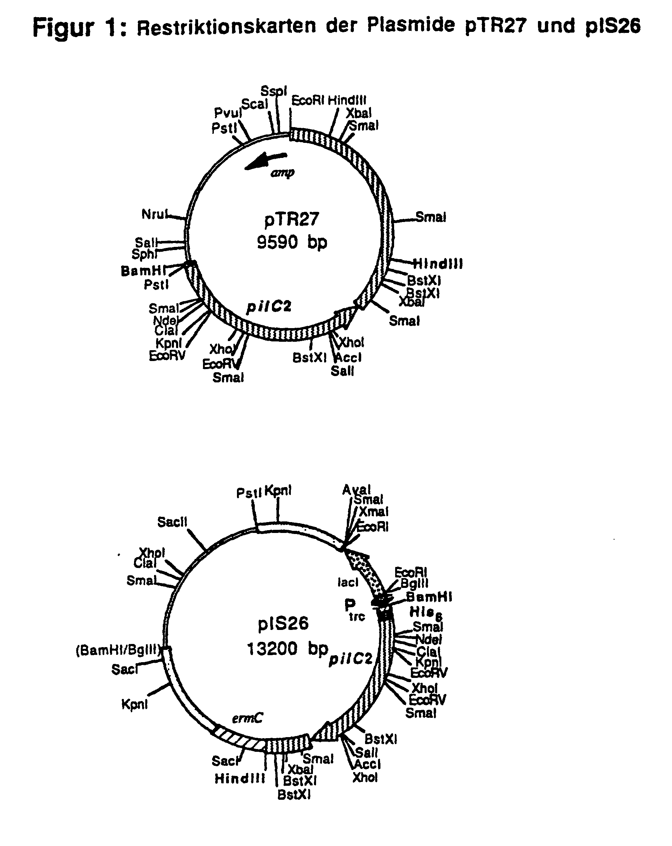

Konstruktionsschema der rekombinanten Gensequenz zur. Synthese des erfindungsgemäßen PilC-Proteins. Die Beschreibung der durchgeführten Arbeiten ist in Beispiel 2 enthalten. Die in der Figur dargestellte DNA-Region entspricht dem 5'-terminalen Bereich des in pTR27 enthaltenen pilC2 Gens (vgl. Figur 1). Die Bezeichnungen "TR..." beziehen sich auf die verwendeten Oligonucleotide (Beispiel 2). (A) Modifikation des phasenvariablen Signalpeptid-codierenden, eine homopolymere Nucleotidsequenz enthaltenden Sequenzabschnitts. (B) Insertion einer His₆-Peptid codierenden Nucleotidsequenz. (C) Erläuterungen zu (A und B).

### Figur 3:

Analytische Gelelektrophorese von gereinigten Pili des N. gonorrhoeae Wildtyp Stammes N137 (1) und der PilC-negativen N. gonorrhoeae Doppelmutante N474 (2), sowie von dem erfindungsgemäßen biologisch aktiven PilC2-Protein (3). Die Isolierung der Pili erfolgte nach Jonsson et al. (1991); die Gewinnung des erfindungsgemäßen PilC2-Proteins ist in Beispiel 3 beschrieben.

### Figur 4:

Nucleotidsequenzen der natürlichen pilC-Gene. (A) Vergleich der Nucleotidsequenzen der Gene pilCl und pilC2 von Neisseria gonorrhoeae MS11-N133; (B) Vergleich der Nucleotidsequenzen des pilC1-Gens von Neisseria gonorrhoeae MS11-N133 mit der partiellen Gensequenz eines pilC-Gens von Neisseria meningitidis A1493; (C) Vergleich der Nucleotidsequenz des pilC2-Gens von Neisseria gonorrhoeae MS11-N133 mit der partiellen Gensequenz eines pilC-Gens von Neisseria meningitidis A1493. Beispiele erfindungsgemäßer konservierter Bereiche sind durch Sterne markiert. Die für die Konstruktion der erfindungsgemäßen rekombinanten Gensequenz vorgenommenen Veränderungen sind in Figur 2 dargestellt.

Die Beispiele erläutern die Erfindung.

### Beispiel 1: Isolierung des pilC2-Gens von N. gonorrhoeae

Anhand der bei Jonsson et al. (*EMBO J*., 10; 477-488, 1991) veröffentlichten Teil-Sequenzen von pilC1 und pilC2, wurden spezifische Oligonucleotid-Sonden entworfen (für pilC1: CG31 CGATGGCGCAAACCCATCAA; für pilC2: CG32 CGCAGGCGCAAACCCGTAAA), mit deren Hilfen beide pilC-Gene aus einer Plasmid-Genbank genomischer DNA von Neisseria gonorrhoeae MS11 isoliert werden konnten. Dafür wurden beide Sonden mit DNA-Kinase am 5'-Ende radioaktiv markierte und gegen ca. 30000 Clone der Genbank hybridisiert. Positive Clone wurden vereinzelt, erneut gegen die radioaktiv markierten Sonden hybridisiert und schließlich charakterisiert. Eines der erhaltenen Plasmide (pTR27, vg. Figur 1) enthielt das gesamte pilC2-Gen, dem allerdings der Promotor zur Synthese von PilC-Protein fehlte. Das pTR27 stellt das Basiskonstrukt dar zur Bestimmung der DNA-Sequenz des pilC2-Gens und zur gentechnischen Modifizierung der DNA- und Protein-Sequenz (vgl. Beispiel 2 und Beispiel 6).

### Beispiel 2: Konstruktion eines PilC-protein überproduzierenden N. gonorrhoeae Stammes

Die Modifikation der homopolymeren Nucleotidsequenz, die im Fall des clonierten pilC2-Gens aus 13 G-Nucleotiden bestand, wurde durch gezielte Mutagenese mit Hilfe der Polymerase-Ketten Reaktion (PCR) in zwei Stufen erreicht. Das Plasmid pTR27 (vgl. Beispiel 1, Figur 1) - diente als Matrize in diesen Reaktionen. Die erste Stufe (1. PCR) umfaßte zwei, separate PCR-Reaktionen des pilC2-Gens mit den Primer-Paarungen TR12 (TTGGATCCGACGTCCGAAAAGGAAATACGATG) und TR28, (GCTCCACCACCTCCGCCGGTATGGGAAAAC), sowie mit TR27 (ACCGGCGGAGGTGGTGGAGCGCAGGCGCAAACCCGT) und TR23 (TGTGTCTCTGCATATGACG) (Figur 1). Die PCR-Reaktionen (25 Zyklen von je 1 Min. 94°C, 2 Min. 50°C und 1 Min. 72°C) wurden in einem Perkin Elmer Cetus Thermal Cycler durchgeführt und enthielten je 100 pMol Primer, ca. 1 ng pTR27 und 2 Einheiten VentR™ Polymerase (New England Biolabs) in einem Volumen von 100 µl. Das Oligonucleotid TR28 hybridisierte direkt vor, das Oligonucleotid TR27 direkt nach dem homopolymeren Nucleotidseguenz-Bereich, was dazu führte, daß weder das erste, noch das zweite PCR-Fragment den homopolymeren Nucleotidsequenz-Bereich enthielten. Stattdessen enthielten die Fragmente 1 und 2 aufgrund der 5'-Erweiterungen der Oligonucleotide TR28 und TR27 eine invariable, heteropolymere Nucleotidsequenz, die für dieselben Aminosäuren codiert wie die ursprüngliche homopolymere Nucleotidsequenz. Die Fragmente 1 und 2 der ersten PCR wurden durch Agarose-Gel-Elektrophorese. aufgetrennt und mit GENECLEAN (BIO 101) extrahiert. Die gereinigten Fragmente wurden in der 2. PCR-Reaktion zu einem, Fragment zusammengefügt, indem jetzt in einer Reaktion mit den Oligonucleotiden TR12 und TR23 amplifiziert wurde (Figur 2). Die Bedingungen der 2. PCR wurden wie folgt gewählt: 100 pMol Primer, Fragment 1 und Fragment 2 in äquimolaren Mengen (je 50 ng) und 2 Units der VentR™ Polymerase. Im Unterschied zur 1. PCR wurden bei der 2. PCR 15 Zyklen unter den gleichen Bedingungen durchgeführt. Der korrekte Austausch der variablen homopolymeren Nucleotidsequenz gegen eine invariable, heteropolymere Nucleotidsequenz wurde durch Analyse der DNA-Sequenz überprüft.
Das erhaltene PCR-Produkt wurde als BamHI/NdeI geschnittenes Fragment gegen das entsprechende BamHI/NdeI Fragment des Vektors pTR27 ausgetauscht, woraus sich Plasmid pTR81 ergab. Um das PilC-Protein über Nickel-Chelat-Affinitätschromatographie reinigen zu können (Hochuli et al., Journal of Chromatography, 411, 177-184, 1987; Hochuli et al., Bio/Technology 6, 1321-1325, 1988), wurde die codierende DNA-Sequenz für ein sechs Histidin Reste umfassendes Peptid (His₆) in das pilC-Gen eingefügt. Die Clonierung der His₆-codierenden DNA-Sequenz wurde analog der oben beschriebenen Technik der gezielten PCR Mutagenese durchgeführt (Fig. 1). Das Plasmid pTR81 diente bei der ersten PCR als Matrize. In getrennten Reaktionen wurden das Fragment 1 mit der Oligonucleotid-Paarung TR12 und TR71 (GTGATGATGGTGGTGATGGGTTTGCGCCTGCGCTCCA) und das Fragment 2 mit der Oligonucleotid-Paarung TR23 und TR70 (CATCACCACCATCATCACCGTAAATACGCTATTATC) amplifiziert. Das Oligonucleotid TR71 paarte mit dem Codon für Thr₃₅ beginnend am (-)-Strang; das Oligonucleotid TR70 paarte mit dem Codon für Arg₃₆ beginnend am (+)-Strang (vgl. Fig. 2). Aufgrund ihrer 5'-Erweiterungen codierten die beiden Oligonucleotide für die His₆-Sequenz, die auf dem Wege der zweiten PCR zwischen die Codons Thr₃₅ und Arg₃₆ in pilC2 eingefügt wurden (vgl. Fig. 2). Das BamHI/NdeI geschnittene PCR-Produkt der 2. PCR wurde gegen das entsprechende BamHI/NdeI Fragment von pTR27 ausgetauscht (vgl. Beispiel 1). Das resultierende Konstrukt wird mit plS25 bezeichnet. Die DNA Sequenzanalyse bestätigte die korrekte Insertion des His₆-Peptids zwischen die Aminosäuren Thr₃₅ und Arg₃₆.

Die induzierbare überexpression des pilC2-Gens mit der nicht-phasenvariablen Nucleotidsequenz und His₆-codierender Nucleotidsequenz wurde erreicht, indem das BamHI/HindIII-Fragment aus dem plS25 in den Hermes-8 Vektor (Kupsch et al., zur Publikation eingereicht) unter Kontrolle des P_{trc}-Promotors zunächst in E. coli K12 cloniert wurde (plS26, vgl. Fig. 1), womit die Isopropyl-ß-D-thiogalactosid (IPTG)-induzierbare Expression des rekombinanten pilC2-Gens unter. Kontrolle des P_{trc} Promotors erreicht wurde. Durch Transformation von N. gonorrhoeae N219 und Allelic Exchange wurde die Shuttle-Box von plS26 mit dem rekombinanten pilC₂-Gen in das konjugative Plasmid ptetM25.2 eingebaut. Hierdurch wurde erreicht, daß das rekombinante Gen in den Pilin-freien N. gonorrhoeae Stamm N174, der aufgrund der Deletion des für Pilin codierenden pilE-Gens nicht transformierbar ist, konjugativ transferiert werden konnte. Der resultierende N. gonorrhoeae Stamm ISN19 synthetisiert nach Induktion mit IPTG in großen Mengen Pilin-freies PilC₂-Protein.

### Beispiel 3: Isolierung von Pilin-freien, biologisch aktivem PilC-Protein

Zur Gewinnung des erfindungsgemäßen, biologisch aktiven. PilC-Proteins wurde der Stamm ISN19 auf 30 kleine GC-Agar Platten (9 cm Durchmesser), die zusätzlich 5 µg/ml Tetracyclin und 100 µM IPTG enthielten, ausgestrichen und 20 h bei 37°C, 5 %CO₂ inkubiert. Die GC-Agar Platten enthielten die für das Wachstum von N. gonorrhoeae notwendigen, Substanzen, insbesondere 36 g GC-Agar-Base (Becton, Dickinson & Company) in 1 1 Wasser autoklaviert und anschließend mit 10 ml steril-filtriertem Vitamin-Mix versetzt. Der Vitamin-Mix enthielt insbesondere 0,01 g Vitamin B12, 1 g Adenin, 0,03 g Guanin, 10 g Glutamin, 0,1 g Cocarboxylase, 0,03 g Thiamin, 25,9 g L-Cystein, 1,1 g L-Cystin, 150 mg Arginin, 0,5 g Uracil, 0,02 g Fe(NO₃)₃, 0,25 Diphosphopyridinnucleotid, 0,013 g p-Aminobenzoesäure und 100 g Dextrose in 1 1 Wasser gelöst. Die Bakterien wurden mit Wattestäbchen in 30 ml 50 mM TRIS.Cl, 0,15 M NaCl, pH 8,0 resuspendiert und bei 4000 Umdrehungen pro Minute (Upm) und 4°C für 15 Min. in einem SS34-Rotor (Sorval) zentrifugiert. Das Pellet wurde in 30 ml 50 mM TRIS.Cl, 0,15 M NaCl, pH 8,0 resuspendiert und die Bakterien durch Zugabe von einer Spatelspitze Lysozym und 5 mM Ethylendiamintetraessigsäure-Dinatriumsalz-Dihydrat (EDTA) durch Ultraschall-Behandlung aufgeschlossen. Die lysierten Bakterien wurden bei 5000 Upm für 20 Min. bei 4°C in dem SS34-Rotor pelletiert. Aus dem resultierenden überstand wurden die Membranen bei 20000 Upm und 4°C für 60 Min. im SS34-Rotor pelletiert und anschließend in 10 ml 50 mM TRIS.Cl, 0,15 M NaCl, 10 % Glycerin, 10 mM MgCl₂, pH 8,0 und 2 % Triton X100 aufgenommen. Die Suspension wurde 45 Min. bei 37°C inkubiert und wiederum bei 20000 Upm und 4°C für 60 Min. zentrifugiert. Das Membranpellet, das vorwiegend aus äußerer Membran bestand, wurde einmal mit 10 ml 50 mM TRIS.Cl, 0,15 M NaCl, pH 8,0 gewaschen und erneut bei 20000 Upm für 60 Min. pelletiert. Nun wurde das Pellet in 10 ml 50 mM TRIS.Cl, 0,15 M NaCl, 10 mM MgCl₂, 10 % Glycerin, pH 8,0 und 2 % LDAO (N,N-Dimethyldodecylamin-N-oxid) suspendiert und für 60 Min. bei 37°C inkubiert. Nach Zentrifugation bei 20000 Upm (4°C) für 60 Min. in dem SS34-Rotor wurde der überstand, in dem das biologisch aktive PilC-Protein gelöst vorliegt, durch Nickel-Chelat-Affinitätschromatographie aufgereinigt. Hierfür wurde eine Nickel-NTA (Ni-Nitrilotriessigsäure)-Agarose-Säule mit 300 µl Säulenvolumen vorbereitet, mit 5 Volumen Aqua bidest. gewaschen und 10 ml des überstandes mit dem extrahierten PilC-Protein aufgetragen. Durch Waschen der Säule mit 300 µl 50 mM TRIS.Cl, 50 mM Imidazol, 10 % Glycerin, pH 8,0 wurden an die Nickel-NTA-Agarose-Säule unspezifisch gebundene Proteine entfernt. Das biologisch aktive PilC-Protein wurde nach Waschen der Säule mit 5-10 Volumen 20 mM NaₓHₓPO₄, 150 mM NaCl, pH 7,5 (PBS), mit einem Citrat/Phosphat-Puffer (10 mM Citronensäure, 1 M NaₓHₓPO₄, pH 3,5, 10 % Glycerin, 0,15 M NaCl) eluiert. Das Eluat wurde unmittelbar mit einer 1 M Na₂HPO₄-Lösung neutralisiert. Das im Eluat in reiner Form enthaltene erfindungsgemäße PilC-Protein wird in flüssigem Stickstoff schockgefroren und bei -70°C gelagert.

### Beispiel 4: Nachweis eines Rezeptors für PilC auf menschlichen Epithelzellen

Pili-gekoppelte fluoreszierende MX Covashere Partikel (FMP) wurden wie folgt hergestellt: FMPs (Duke Scientific Corporation, Paolo Alto, California, USA) besitzen eine aus aktiven Gruppen bestehende Oberfläche, die eine direkte, spontane Kopplung von Proteinen ermöglicht. Im allgemeinen wurde 100 µl der FMPs mit einem Durchmesser von 0,5 µm mit ca. 2 µg gereinigten Pili (nach Jonsson et al., 1991) in 100 µl PBS versetzt und für 2 h bei Raumtemperatur auf einer Rotationsscheibe gemischt. Die mit Pili beschichteten Partikel wurden pelletiert und in 1 ml Blockierungspuffer (20 mM TRIS. Cl mit 2 % Fetuin, pH 7,5) gewaschen, um die, freien Kopplungsgruppen zu blockieren. Die Proteinkonzentration im überstand nach der ersten Zentrifugation wurde mit der eingesetzten Pilimenge verglichen, um die Ausbeute der Kopplung zu bestimmen. Mehr als 80 **%** der eingesetzten Pili wurden bei dieser Reaktion kovalent an FMPs gebunden. Die Partikel wurden erneut pelletiert und in 100 µl PBS aufgenommen. Die FMPs wurden sowohl mit gereinigten Pili eines adhärenten Neisseria gonorrhoeae Wildtyp Stammes (FMP-Pilin/PilC), als auch mit gereinigten Pili einer pilCl/2 Deletionsmutante (MS11-N474: Facius et al., zur Publikation eingereicht) (FMP-Pilin) und mit Fetuin (FMP-Fetuin: negativ Kontrolle) beschichtet. Zum Nachweis von PilC-Protein-spezifischen Rezeptoren auf menschlichen Epithelzellen wurde die Bindung von Pilibeschichteten FMPs an Epithelzellinien untersucht. Diese, Experimente wurden analog dem Standard-Infektionsprotokoll mit Gonokokken durchgeführt. Epithelzellen wurden in 24-Well-Zellkultur-Platten auf sterilen Deckgläschen in RPMI Medium mit 5 % fötalem Kälberserum (FCS) bei 37 °C und unter 5 % CO₂-Begasung kultiviert. Präkonfluente Epithelzell-Monolayer wurden mit frischem Medium gewaschen bevor je Well 10 µl der Pili-beladenen FMP-Suspension zugesetzt wurde. Die Adhärenzdauer betrug 1 h, währenddessen die Kulturen bei 37°C und 5 % CO₂ inkubiert wurden. Dann wurden ungebundene FMPs durch 5-maliges Waschen mit PBS entfernt und mit 2 % Paraformaldehyd in PBS für 30 Min. fixiert. Die Präparate konnten anschließend unter einem Zeiss-Fluoreszenz-Mikroskop betrachtet werden. Die in Tabelle 1 zusammengestellten Ergebnisse dieses Versuchs zeigen, daß im Gegensatz zu den Wildtyp-Pili die PilC-freien Pili keine Bindung an Epithelzellen vermitteln. Die Bindung von PilC-freien Pili an Epithelzellen kann jedoch komplementiert werden durch Zugabe von dem erfindungsgemäßen biologisch aktivem PilC2-Protein. Der Zugabe von 2 µg biologisch aktivem PilC2-Protein in 100 µl PBS zu 100 µl der bereits beschichteten FMP-Pilin folgte eine 2 h Inkubation bei 20 °C unter ständiger Durchmischung. Die Partikel wurden pelletiert, in 1 ml PBS gewaschen und in 100 µl PBS aufgenommen. Die nachträglich mit PilC-Protein beschichteten FMP-Pilin werden mit FMP-(Pilin + PilC) bezeichnet.

**Tabelle 1:**

| Bindung fluoreszierender Partikel an menschliche Epithelzellen | |
|---|---|
| FMPs | FMPs pro Epitheizelle |
| FMP-Fetuin | 0.1 - 3 |
| FMP-Pilin/PilC | 150-400 |
| FMP-Pilin | 0.1-5 |
| FMP-(Pilin + PilC) | 100-300 |

Bindung fluoreszierender Partikel (FMPs) an menschliche Epithelzellen. FMP-Fetuin, Fetuin gekoppelte FMPs; FMP-Pilin/PilC, mit gereinigten Pili des N. gonorrhoeae Stammes N137 gekoppelte FMPs; FMP-Pilin, mit gereinigten Pili des N. gonorrhoeae Stammes N474 gekoppelte FMPs; FMP- Pilin + PilC, mit gereinigten Pili des N. gonorrhoeae Stammes N474 gekoppelte FMPs, die nachträglich mit erfindungsgemäßem PilC-Protein inkubiert wurden (Beispiel 4). Die Ergebnisse der Bindung sind in der durchschnittlichen Anzahl der FMPs pro Epithelzelle angegeben.

### Beispiel 5: Direkter Nachweis der Bindung von PilC an Rezeptoren auf menschlichen Epithelzellen

Zum Nachweis von PilC-Protein-spezifischen Rezeptoren auf menschlichen Epithelzellen wurde die Bindung von erfindungsgemäßem biologisch aktivem PilC-Protein an ME180-Zellen (human cervix carcinoma (ATCC HTB33» untersucht. Die Epithelzellen wurden in 4 Well Chamber Slides ™ (Nunc) in RPMI Medium mit 5 % fötalem Kälberserum (FCS) bei 37°C, 5 % CO₂ kultiviert. Der Zugabe von ca. 10 µg biologisch aktivem PilC-Protein in einem maximalen Volumen von 20 µl folgte eine 30 Min. Inkubation bei 37°C und 5 % CO₂. Jedes Well wurde dreimal mit 1 ml PBS gewaschen, anschließend wurden Epithelzellen und gebundenes PilC-Protein für 30 Min. in 2 % Paraformaldehyd in PBS fixiert. Um gebundenes PilC-Protein sichtbar zu machen, wurde eine Immun-Fluoreszenz Färbung mit spezifischen Antiseren gegen das erfindungsgemäße biologisch aktive PilC-Protein durchgeführt. Diese Antiseren wurden. durch Immunisierung von Balb/c Mäusen mit dem erfindungsgemäßen biologisch aktiven PilC-Protein erhalten. Die fixierten Präparate wurden zweimal mit 1 ml PBS gewaschen und mit einer 1:300 Verdünnung der Antiseren in PBS für die Dauer von 1 h inkubiert. Dann wurde fünfmal mit PBS, 0,05 % Tween20 gewaschen, um unspezifisch gebundene Antikörper (AK) zu entfernen. Um den PilC-AK-Komplex sichtbar zu machen, wurde mit einem zweiten AK, der gegen Mäuse-Immunglobuline gerichtet war und mit dem Fluoreszenz-Farbstoff FITC markiert war, in einer 1:2000 Verdünnung in PBS mit 0,025 % Tween20 für 60 Min. bei 20°C inkubiert. Wieder wurde sorgfältig mit PBS, 0,05 % Tween20 gewaschen (5 mal), bevor die Präparate eingedeckelt und unter einem Zeiss-Fluoreszenz-Mikroskop ausgewertet wurden. Als Negativkontrollen dienten (a) Epithelzellen ohne PilC mit 2. AK; (b) Epithelzellen ohne PilC mit 1. AK und 2. AK, und (c) Epithelzellen mit PilC und 2. AK. Während die mit dem erfindungsgemäßen biologisch aktiven PilC-Protein vorinkubierten Epithelzellen nach Anfärben mit spezifischen PilC-Antiseren stark fluoreszierten, konnte bei allen Kontrollexperimenten nur eine schwache Fluoreszenz beobachtet werden. Als weitere Kontrolle wurde das Experiment mit MDCK-Zellen durchgeführt, an die pilierte Neisserien nicht binden; entsprechend konnte auch keine Bindung des erfindungsgemäßen PilC-Proteins nachgewiesen werden.

### Beispiel 6: Kompetition der Infektion von menschlichen Epithelzellen mit Neisserien

Die Durchführung der in vitro-Infektions- bzw. Inhibitionsexperimente erfolgte mit präkonfluenten ME180-Zellen [human cervix carcinoma (ATCC HTB33)], die auf Deckgläser in einer 24-Well Zellkulturplatte kultiviert wurden. Zur Inhibition der Pilus-vermittelten Adhärenz von N. gonorrhoeae und N. meningitidis wurden die Zellen in 500 *µ*l RPMI-Medium, 5 % FCS durch Zugabe von 20 µg des biologisch aktiven PilC-Proteins 30 Min. bei 37°C und 5 % CO₂ inkubiert. Zur Infektion wurden die Bakterien auf einer GC-Platte über Nacht bei 37°C, 5 % CO₂ angezogen und dann mit einem Wattestäbchen in 1 ml RPMI-Medium, 5 % FCS resuspendiert. Die Bestimmung der Bakteriendichte erfolgte mit dem Spektralphotometer. Die etwa 2x10⁵ präkonfluenten Epithelzellen wurden mit 7x10⁷ Bakterien infiziert und bei 37°C, 5 % CO₂ 1 h lang inkubiert. Das Abstoppen der Infektion erfolgte durch fünfmaliges Waschen mit vorgewärmtem PBS und anschließendem Fixieren der Zellen mit 2 % Paraformaldehyd in PBS für 30 Min. Die fixierten Zellen wurden anschließend für mindestens 15 Min. bei Raumtemperatur mit Kristallviolett (0,07 % w/v) gefärbt und die adhärenten Bakterien pro ME-180 bestimmt. Wie Tabelle 2 zeigt, adhärierten in Abwesenheit von dem biologisch aktiven PilC2-Protein alle getesteten piliierten Neisseria-Stämme, während der nicht-piliierte N. gonorrhoeae Stamm nicht adhärierte. In Gegenwart von dem biologisch aktiven PilC2-Protein wurde die Bindung der getesteten piliierten Neisseria-Stämme auf Hintergrund-Niveau reduziert. Die Reduktion der Bindung an Epithelzellen erfolgte somit unabhängig von der Variante des von den Bakterien natürlich gebildeten PilC-Proteins (PilC1 in TRN289, gegenüber PilC2. in TRN290, gegenüber einem unbekannten PilC-Protein in N. meningitidis N530). Damit wird (i) der Nachweis des PilC-Proteins als wichtiges (d.h. für das Zustandekommen einer Infektion essentielles) bakterielles Adhäsin geführt, (ii) die geringe Variabilität unterschiedlicher PilC-Proteine in bezug auf die Erkennung von zellulären Rezeptoren bestätigt. und daher (iii) die Eignung der erfindungsgemäßen Inhibitoren der Wechselwirkung zwischen PilC-Proteinen und. seinen zellulären Rezeptoren belegt.

**Tabelle 2:**

| Kompetition der Bindung von Neisserien an menschliche Epithelzellen | | |
|---|---|---|
| Neisserien Stämme | Neisserien pro Epithelzelle | |
| | Vorbehandlung der Epithelzellen | |
| | ohne PilC | 20 µg/ml PilC |
| N137 | 165 | - |
| N174 | - | - |
| TRN289 (pilE_{N137;} PilC1) | 140 | - |
| TRN290 (pilE_{N137}; PilC2) | 155 | - |
| N530 | 103 | - |

Kompetition der Bindung von pathogenen Neisserien an menschliche Epithelzellen. N137, piliierter N. gonorrhoeae MS11 Stamm; N174, von N. gonorrhoeae MS11 abgeleiteter nicht-piliierter Stamm, in dem das pilE-Gen deletiert ist; TRN289, piliierter N. gonorrhoeae MS11 Stamm, der ausschließlich PilC1 bildet; TRN290, piliierter N. gonorrhoeae MS11 Stamm, der ausschließlich PilC2 bildet, und N530, N. meningitidis Stamm, der ein unbekanntes PilC-Protein bildet. Die Vorbehandlung der Epithelzellen mit 20 µg/ml erfindungsgemäßem PilC2-Protein ist in Bespiel 6 beschrieben. Die Ergebnisse des Kompetitionsexperiments sind in der durchschnittlichen Anzahl adhärenter Bakterien pro Epithelzelle angegeben.

### Beispiel 7: Identifizierung weiterer PilC-Protein-codierender DMA-Sequenzen

Zur Bestimmung der DNA-Sequenz des pilC2-Gens wurde das BamHI/EcoRI-Fragment des pTR27 in den Blueskript SK-Vektor cloniert (pTR34). Mit der Exonuclease III wurden geeignete, überlappende Deletionsclone vom pTR34 erzeugt und sequenziert. Beim Vergleich der für das PilC1 (A. Jonsson, Umea University, New Series No. 322, Department of Microbiology; ISSN 0346-6612) und das PilC2-Protein codierenden DNA-Sequenzen (Figur 4) mit dem Computerprogramm PCGENE/ALGIN ergab sich eine 84 %ige Identität. Die Bereiche identischer Sequenzen verteilen sich inselartig über beide Gene mit einer eindeutigen Konzentrierung am 3'-Ende. Mit der Absicht, weitere für PilC-Protein codierende Sequenzen zu identifizieren, wurden Oligonucleotid-Sonden für die zwischen pilC1 und pilC2 identischen Sequenzen entworfen. Besonderer Wert wurde beim Entwurf der Oligonucleotid-Sonden darauf gelegt, die Oligonucleotide abwechselnd von DNA (+) und (-) Strang abzuleiten, um bei Bedarf entsprechende Fragmente durch PCR amplifizieren zu können. Die gesamte DNA-Sequenz der pilC1 und pilC2-Gene wurde so in überlappende, ca. 400-500 bp-Fragmente aufgeteilt. Außerdem wurde jedem (+)-Strang-Oligonucleotid die "M13mp" Primer, jedem (-)-Strang-Oligonucleotid die "M13mp reverse" Primer (Vieira und Messing, Gene 19, 259-268, 1982) Hybridisierungssequenz ans 5'-Ende angefügt, um die DNA-Sequenz der PCR-Fragmente direkt bestimmen zu können.
Eine Auswahl von Oligonucleotid-Sonden wurde nach Vorschrift des DIG-Oligonucleotide-Tailing-Kits (Boehringer Mannheim) mit Biotin markiert und zur Hybridisierung gegen mit ClaI und PvuII geschnittener chromosomaler DNA von N. gonorrhoeae eingesetzt. Im Southern-Blott ergaben sich unter Verwendung der genannten Oligonucleotid-Sonden (Tabelle 3) eindeutige Hinweise auf die Existenz weiterer pilC-Gene (neben pilC1 und pilC2) im N. gonorrhoeae Stamm MS11 und im N. meningitidis Stamm A1493. Darüber hinaus wurden mit diesen Sonden Gene für PilC-analoge Proteine in einem Pseudomonas aeruginosa Stamm nachgewiesen. Die so identifizierten Gene können nun mittels Standard-Techniken cloniert, charakterisiert und weiterbearbeitet werden. Mit diesem Experiment wurde die Eignung der beschriebenen Methode für die Identifizierung bisher unbekannter Gene für PilC- bzw. PilC-analoge Proteine nachgewiesen.

**Tabelle 3:**

| Oligonucleotid-Sonden zur Identifizierung weiterer Pilc-Protein-Codierender Nucleotidsequenzen | | | |
|---|---|---|---|
| Oligonukleotid | 5' Position pilC1/pilC2 | Sequenz | +/- DNA Strang |
| TR47 | 117/117 | TATTATCATGAACGAGCG | + |
| TR48 | 392/413 | CGGGTGGTACGAATCCAA | - |
| TR49 | 322/343 | AAGGTTTCCGGTTTGATG | + |
| TR50 | 692/713 | ACGATGGTTTGATTATTA | - |
| TR51 | 590/611 | GCGTATCTTTCAATTTGG | + |
| TR52 | 1033/1060 | ACCACAGCGCGGCGGCGGTCAG | - |
| TR53 | 935/965 | AGTCAAAGCAGGCCGCTG | + |
| TR54 | 1411/1423 | CCGTCCAAGGCAGCAGCAC | - |
| TR55 | 1327/1339 | AAAAACGACACTTTCGGC | + |
| TR56 | 1757/1799 | TCCACGCCGTAGCGGTCG | - |
| TR57 | 1630/1672 | AATCTGAAGCTCAGCTAC | + |
| TR58 | 2015/2072 | AGGAAGGCGGCGTATITG | - |
| TR59 | 1957/2016 | TACACCGTCGGTACGCCGC | + |
| TR60 | 2329/2386 | CTGCCAGTCGGGAAACGGC | - |
| TR61 | 2253/2311 | TAGTAAATGGTCTGCAAAG | + |
| TR62 | 2611/2674 | TACGCAATACCACGGTCG | - |
| TR63 | 2563/2626 | TTGAGGGAAGGAGAACGCG | + |
| TR64 | 2878/2941 | CCAG(G/A)TAACGCACATTAACC | - |
| TR65 | 2823/2886 | AACCGTCTGCCCGAACGG | + |
| TR66 | | TTCGGACGGCATTTGCGG | - |

Beispiele von oligonucleotid-Sonden zur Identifizierung weiterer PilC-Protein-codierender Nucleotidsequenzen. Die Beispiele TR47-TR65 sind dem Sequenzvergleich der N. gonorrhoeae-Gene pilC1 und pilC2 in Figur 4A entnommen. TR66 entspricht einem Sequenzbereich stromabwärts von dem codierenden Bereich der pilC-Gene. Die Positionsnummern beziehen sich auf die pilC1 bzw. pilC2-Gene in Figur 4A.

## Patentansprüche

1. Rekombinante Gensequenz zur Synthese eines Proteins mit der biologischen Aktivität des PilC-Proteins, wobei die Aktivität darin besteht, dass das Protein das Assembly von Typ 4-Pili unterstützt, die Bindung von Typ 4-Pilus-tragenden Bakterien an zelluläre Rezeptoren vermittelt oder die immunologische Eignung zur Induktion von Antikörpern- gegen Typ 4-Pilus-tragende Bakterien aufweist, die mit der Anlagerung der Bakterien an ihre zellulären Rezeptoren kompetieren und vorzugsweise die Anlagerung blockieren, deren phasenvariabler Signal peptidcodierender, eine homopolymere Nucleotidsequenz enthaltender Sequenzabschnitt durch die folgende Veränderung charakterisiert ist,
(a) Modifikation der homopolymeren Nucleotidsequenz zu einer invariablen heteropolymeren Nucleotidsequenz,
so dass die Expression der rekombinanten Gensequenz in einer Wirtszelle unbeeinflusst von Phasenvariation ermöglicht wird.

2. Gensequenz nach Anspruch 1, erhältlich durch Modifikation einer aus einem pathogenen, Typ 4-Pilus-tragenden Bakterium stammenden DNA-Sequenz.

3. Gensequenz nach Anspruch 2, wobei das pathogene Bakterium ein Bakterium der Gattung Neisseria, vorzugsweise Neisseria gonorrhoeae oder Neisseria meningitidis ist.

4. Gensequenz nach einem der Ansprüche 1 bis 3, die die in der Figur 4 dargestellte Gensequenz ist, wobei die homopolymere, phasenvariable Nucleotidsequenz durch die in Fig. 2 dargestellte heteropolymere, nichtphasenvariable Nucleotidsequenz ersetzt ist.

5. Gensequenz nach einem der Ansprüche 1 bis 4, erhältlich durch Modifikation einer DNA-Sequenz, die mit einem konstanten Bereich einer der DNA-Sequenzen der Figur 4 bzw. Tabelle 3 hybridisiert und ein Protein mit der biologischen Aktivität des PilC-Proteins codiert, wobei die Aktivität darin besteht, dass das Protein das Assembly von Typ 4-Pili unterstützt, die Bindung von Typ 4-Pilus-tragenden Bakterien an zelluläre Rezeptoren vermittelt oder die immunologische Eignung zur Induktion von Antikörpern gegen Typ 4-Pilus-tragende Bakterien aufweist, die mit der Anlagerung der Bakterien an ihre zellulären Rezeptoren kompetieren und vorzugsweise die Anlagerung blockieren.

6. Gensequenz nach einem der Ansprüche 1 bis 5, wobei die zu einer heteropolymeren Nucleotidsequenz modifizierte homopolymere Nucleotidsequenz mindestens 5 Nucleotide enthält.

7. Gensequenz nach einem der Ansprüche 1 bis 6, die ein Protein mit der biologischen Aktivität des PilC-Proteins codiert, das einen zu seiner Reinigung geeigneten Oligo-Histidin-Bereich aufweist, wobei die Aktivität darin besteht, dass das Protein das Assembly von Typ 4-Pili unterstützt, die Bindung von Typ 4-Pilus-tragenden Bakterien an zelluläre Rezeptoren vermittelt oder die immunologische Eignung zur Induktion von Antikörpern gegen Typ 4-Pilus-tragende Bakterien aufweist, die mit der Anlagerung der Bakterien an ihre zellulären Rezeptoren kompetieren und vorzugsweise die Anlagerung blockieren.

8. Gensequenz nach Anspruch 7, wobei sich der Oligo-Histidin-Bereich am N-Terminus oder am C-Terminus der reifen Form des codierten Proteins befindet.

9. Rekombinanter Vektor, der eine Gensequenz nach einem der Ansprüche 1 bis 8 enthält.

10. Rekombinanter Vektor nach Anspruch 9, in dem die genannte Gensequenz unter der Kontrolle eines Promotors steht.

11. Rekombinanter Vektor nach Anspruch 10, in dem der Promotor ein in Neisseria-Wirtszellen aktiver Promotor ist.

12. Wirtszelle, die einen rekombinanten Vektor nach einem der Ansprüche 9 bis 11 enthält.

13. Wirtszelle nach Anspruch 12, die ein nicht piliierter Neisseria-Stamm ist.

14. Verfahren zur Herstellung eines im wesentlichen reinen Proteins mit der biologischen Aktivität des PilC-Proteins, bei dem man eine Wirtszelle nach Anspruch 12 oder 13 unter geeigneten Bedingungen züchtet und das Protein reinigt, wobei die Aktivität darin besteht, dass das Protein das Assembly von Typ 4-Pili unterstützt, die Bindung von Typ 4-Pilus-tragenden Bakterien an zelluläre Rezeptoren vermittelt oder die immunologische Eignung zur Induktion von Antikörpern gegen Typ 4-Pilus-tragende Bakterien aufweist, die mit der Anlagerung der Bakterien an ihre zellulären Rezeptoren kompetieren und vorzugsweise die Anlagerung blockieren.

15. Verfahren nach Anspruch 14, bei dem die Reinigung eine Affinitätschromatographie beinhaltet.

## Claims

1. A recombinant genetic sequence for synthesizing a protein having the biological activity of the PilC protein, wherein the activity means that the protein supports the assembly of type-4-pili, mediates the binding of bacteria bearing type-4-piti to cellular receptors or exhibits the immunologic suitability for inducing antibodies against bacteria bearing type-4-pili which compete with the attachment of the bacteria to their cellular receptors and preferably block the attachment, whose phase-variable sequence portion encoding the signal peptide and containing a homopolymeric nucleotide sequence is **characterized by** the following modification:
(a) modification of the homopolymeric nucleotide sequence to form an invariable heteropolymeric nucleotide sequence
so that the expression of the recombinant genetic sequence in a host cell is made possible unaffected by phase variations.

2. The genetic sequence according to claim 1, obtainable by modification of a DNA sequence derived from a pathogenic bacterium bearing type-4-pili.

3. The genetic sequence according to claim 2, wherein the pathogenic bacterium is a bacterium of the genus Neisseria, preferably Neisseria gonorrhoeae or Neisseria meningitidis.

4. The genetic sequence according to any one of claims 1 to 3 which is the genetic sequence shown in Figure 4, wherein the homopolymeric, phase-variable nucleotide sequence is replaced by the heteropolymeric, non-phase-variable nucleotide sequence shown in Fig. 2.

5. The genetic sequence according to any one of claims 1 to 4, obtainable by modification of a DNA sequence that hybridizes to a constant region of one of the DNA sequences of Figure 4 or Table 3 and encodes a protein having the biological activity of the PilC protein, wherein the activity means that the protein supports the assembly of type-4-pili, mediates the binding of bacteria bearing type-4-pili to cellular receptors or exhibits the immunologic suitability for inducing antibodies against bacteria bearing type-4-pili which compete with the attachment of the bacteria to their cellular receptors and preferably block the attachment.

6. The genetic sequence according to any one of claims 1 to 5, wherein the homopolymeric nucleotide sequence modified to form a heteropolymeric nucleotide sequence comprises at least 5 nucleotides.

7. The genetic sequence according to any one of claims 1 to 6 that encodes a protein having the biological activity of the PilC protein having an oligo histidine portion suitable for its purification, wherein the activity means that the protein supports the assembly of type-4-pili, mediates the binding of bacteria bearing type-4-pili to cellular receptors or exhibits the immunologic suitability for inducing antibodies against bacteria bearing type-4-pili which compete with the attachment of the bacteria to their cellular receptors and preferably block the attachment.

8. The genetic sequence according to claim 7, wherein the oligo histidine portion is located at the N-terminus or at the C-terminus of the mature form of the encoded protein.

9. A recombinant vector containing a genetic sequence according to any one of claims 1 to 8.

10. The recombinant vector according to claim 9, wherein said genetic sequence is under the control of a promoter.

11. The recombinant vector according to claim 10, wherein the promoter is a promoter active in Neisseria host cells.

12. A host cell containing a recombinant vector according to any one of claims 9 to 11.

13. The host cell according to claim 12 that is a non-piliated Neisseria strain.

14. A method for the production of a substantially pure protein having the biological activity of the PilC protein, wherein a host cell according to claim 12 or 13 is cultivated under suitable conditions and the protein is purified, wherein the activity means that the protein supports the assembly of type-4-pili, mediates the binding of bacteria bearing type-4-pili to cellular receptors or exhibits the immunologic suitability for inducing antibodies against bacteria bearing type-4-pili which compete with the attachment of the bacteria to their cellular receptors and preferably block the attachment.

15. The method according to claim 14, wherein the purification comprises affinity chromatography.

## Revendications

1. Séquence génique recombinante pour la synthèse d'une protéine ayant l'activité biologique de la protéine PilC, l'activité consistant en ce que la protéine promeut l'assemblage de Pili de type 4, intervient dans la liaison de bactéries comportant un Pili de type 4 aux récepteurs cellulaires ou présente l'aptitude immunologique à l'induction d'anticorps contre des bactéries comportant un Pili de type 4, lesquels entrent en compétition avec la fixation des bactéries à leurs récepteurs cellulaires et bloquent préférentiellement la fixation, dont la partie de séquence codant pour un peptide signal à phase variable, et contenant une séquence nucléotidique homopolymère, est **caractérisée par** la modification suivante :
(a) modification de la séquence nucléotidique homopolymère en une séquence nucléotidique invariable hétéropolymère,
de manière à ce que l'expression de la séquence génique recombinante devienne possible dans une cellule hôte sans influence d'une variation de phase.

2. Séquence génique selon la revendication 1, pouvant être obtenue par modification d'une séquence d'ADN pathogène provenant d'une bactérie comportant un Pili de type 4.

3. Séquence génique selon la revendication 2, dans laquelle la bactérie pathogène est une bactérie du genre Neisseria, de préférence Neisseria gonorrhoeae ou Neisseria meningitidis.

4. Séquence génique selon l'une des revendications 1 à 3, qui est la séquence génique représentée à la figure 4, dans laquelle la séquence nucléotidique homopolymère à phase variable est remplacée par la séquence nucléotidique hétéropolymère à phase invariable représentée à la figure 2.

5. Séquence génique selon l'une des revendications 1 à 4, pouvant être obtenue par modification d'une séquence d'ADN, qui hybride avec un domaine constant d'une séquence d'ADN de la figure 4, respectivement du tableau 3 et qui code pour une protéine ayant l'activité biologique de la protéine PilC, l'activité consistant en ce que la protéine promeut l'assemblage de Pili de type 4, intervient dans la liaison de bactéries comportant un Pili de type 4 aux récepteurs cellulaires ou présente l'aptitude immunologique à l'induction d'anticorps contre des bactéries comportant un Pili de type 4, lesquels entrent en compétition avec la fixation des bactéries à leurs récepteurs cellulaires et bloquent préférentiellement la fixation.

6. Séquence génique selon l'une des revendications 1 à 5, dans laquelle la séquence nucléotidique homopolymère modifiée en une séquence nucléotidique hétéropolymère contient au moins 5 nucléotides.

7. Séquence génique selon l'une des revendications 1 à 6, qui code pour une protéine ayant l'activité biologique de la protéine PilC, qui présente un domaine Oligo-histidine approprié à sa purification, l'activité consistant en ce que la protéine promeut l'assemblage de Pili de type 4, intervient dans la liaison de bactéries comportant un Pili de type 4 aux récepteurs cellulaires ou présente l'aptitude immunologique à l'induction d'anticorps contre des bactéries comportant un Pili de type 4, lesquels entrent en compétition avec la fixation des bactéries à leurs récepteurs cellulaires et bloquent préférentiellement la fixation.

8. Séquence génique selon la revendication 7, dans laquelle le domaine Oligo-histidine se trouve à l'extrémité N-terminale ou C-terminale de la forme mature de la protéine codée.

9. Vecteur recombinant, contenant une séquence génique selon l'une des revendications 1 à 8.

10. Vecteur recombinant selon la revendication 9, dans lequel ladite séquence génique est placée sous le contrôle d'un promoteur.

11. Vecteur recombinant selon la revendication 10, dans lequel le promoteur est un promoteur actif dans une cellule hôte Neisseria.

12. Cellule hôte, contenant un vecteur recombinant selon l'une des revendications 9 à 11.

13. Cellule hôte la revendication 12, qui est une souche Neisseria exempte de pili.

14. Procédé de production d'une protéine essentiellement pure ayant l'activité biologique de la protéine PilC, dans lequel on cultive une cellule hôte selon l'une des revendications 12 ou 13 dans des conditions appropriées et on purifie la protéine, l'activité consistant en ce que la protéine promeut l'assemblage de Pili de type 4, intervient dans la liaison de bactéries comportant un Pili de type 4 aux récepteurs cellulaires ou présente l'aptitude immunologique à l'induction d'anticorps contre des bactéries comportant un Pili de type 4, lesquels entrent en compétition avec la fixation des bactéries à leurs récepteurs cellulaires et bloquent préférentiellement la fixation.

15. Procédé selon la revendication 14, dans lequel la purification inclut une chromatographie par affinité.
